# EUROPEAN PATENT SPECIFICATION

(11) **EP 2 141 179 B1**
(45) Date of publication and mention of the grant of the patent: **01.12.2010**
(21) Application number: 08159781.7
(22) Date of filing: 04.07.2008
(51) Int. Cl.: C07K 14/71, C12N 15/62

(54) **A fluorescence based reporter construct for the direct detection of TGF-beta receptor activation and modulators thereof**
Reportergenkonstrukt auf Fluoreszenzbasis zur direkten Erkennung der Aktivierung von TGF-Beta-Rezeptoren sowie Modulatoren dafür
Construction de rapporteur basée sur la fluorescence pour la détection directe de l'activation du récepteur TGF-beta et modulateurs correspondants

(43) Date of publication of application: 06.01.2010
(73) Proprietor: Technische Universität Dresden, 01069 Dresden (DE)
(72) Inventor: Bökel, Christian, Dr., 01259 Dresden (DE); Weidemann, Thomas, Dr., 01277 Dresden (DE)
(74) Representative: Kailuweit, Frank

(56) References cited:
- EP-A- 1 238 982
- WO-A-2005/019447
- US-B1- 6 469 154
- STOCKWELL B R ET AL: "TGF-BETA-SIGNALING WITH SMALL MOLECULE FKBP12 ANTAGONISTS THAT BIND MYRISTOYLATED FKBP12-TGF-BETA TYPE I RECEPTOR FUSION PROTEINS" CHEMISTRY AND BIOLOGY, CURRENT BIOLOGY, LONDON, GB, vol. 5, no. 7, 1 July 1998 (1998-07-01), pages 385-395, XP000865521 ISSN: 1074-5521
- WANG T ET AL: "Specific Interaction of Type I Receptors of the TGF-beta Family with the Immunophilin FKBP-12" SCIENCE, WASHINGTON, DC, vol. 265, 29 July 1994 (1994-07-29), pages 674-676, XP002173914 ISSN: 0036-8075

## Description

### Field of the invention:

This invention relates to a fusion protein acting as sensor for receptor activity and uses thereof, nucleic acid molecules encoding for said fusion protein, a method for detecting receptor activity and screening compounds for modulators of receptor activity using said fusion protein.

### State of the art:

Sensors proteins using circularly permutated green fluorescent protein (cpGFP) as a fusion partner are known from the state of the art. For instance, WO 2005019447A discloses a monochrome fluorescent probe to measure activity of a protein phosphorylation enzyme. The monochrome fluorescent probe comprises cpGFP, a substrate domain and a phosphorylation recognition domain, whereas the substrate domain interacts with the phosphorylation recognition domain when it is phosphorylated. This probe allows to measure a kinase activity in a cell downstream in the signalling pathway, which cannot be allocated to an activity of a specific receptor. Further the probe acts as an artificial substrate and thus competes with endogen substrates, which may lead to dominant negative effects

EP 1238982 A1 and US 6,469,154 disclose indicators for calcium on the basis of calmodulin and fluorescent proteins.

Signalling molecules of the TGF-beta (TGFβ) family (TGF-beta, activins, bone morphogenetic proteins (BMPs) play a role in many biologically and medically relevant processes, e.g. stem cell maintenance, apoptosis regulation, inflammation processes, embryogenesis or cancer.

All TGF-beta family growth factors signal through a heterooligomeric transmembrane receptor consisting of two different Serine/Threonine kinase receptors termed type I and. II receptors (Shi and Massague, 2003). After ligand binding, the type II receptor phosphorylates the type I receptor at its membrane proximal GS domain. This displaces the inhibitor FKBP12 which binds to the nonphosphorylated GS domain of the type I receptor and inactivates its kinase domain. FK506 is a FKBP-12 antagonist that prevents binding to the type I receptor (Wang et al. 1994, Science 256: pp. 674-676).

After phosphorylation the now unmasked GS domain serves as a binding site for the R-SMAD transcription factor (in *Drosophila* called Mad), which is in turn phosphorylated by the type I receptor that has become active after displacement of the inhibitor. The phosphorylated R-SMAD then complexes with cofactors and enters the nucleus. Thus, phosphorylation of the type I receptor is the critical switch between the active and inactive state of the receptor (Huse et al., 2001).

This general order of events holds true for all TGF-beta growth factor subfamilies, i.e. the Bone Morphogenetic Protein (BMP) subfamily, the Activin/Nodal subtype and finally TGF-beta itself. Minor variations in signalling via the different ligand subfamilies concern the order of receptor/ligand and receptor/receptor complex formation, the presence or absence of coreceptors for ligand capture, or the recruitment of the R-Smad transcription factors to the receptor complex by cytoplasmic cofactors leading to a coupling of signalling and endocytosis. However, the actual transition from the inactive to the active state of the receptor is in all cases achieved by phosphorylation of the type I receptor GS domain.

TGF-beta signalling in vertebrates is characterized by a significant convergence of signalling processes along the pathway. In the human genome, there are 42 ORF encoding TGF-beta family growth factors that may form homo- or heterodimers with other members of their ligand subfamily. These are bound by seven type I and five type II receptors. Although these receptors show some ligand subfamily preference, there is a certain level of promiscuity, which is also reflected in the different options for receptor heterodimerization.

The R-SMAD transcription factors that bind to and become phosphorylated by the activated type I receptors exhibit a much higher receptor specificity. Thus, BMP signals are transduced by three highly related R-Smads (Smads 1, 5, and 8) and activin/TGF-beta signals by two (Smad 2 and 3). All five R-Smads bind the same Co-Smad (Smad 4) before shuttling to the nucleus and directing target gene transcription.

Current assays for TGF-beta pathway activation include immunostainings against the phosphorylated R-SMADs, the quantification of endogenous target gene expression levels, or the activity of reporter genes. However, due to the efficient nuclear import of the phospho-SMADs none of these assays impart any information about where on the surface the signal is received. In addition, they only allow discrimination between the two classes of R-Smads involved, but due to the convergence of the upstream signalling pathways they do not give any information on the receptors involved. Further, antibodies against phosphorylated R-SMAD proteins, do not allow observing receptor activity *in vivo.*

Stockwell et al. (1998, Chemistry and Biology, Current Biology 5(7): P. 385-395) measure the activation of TGF-beta receptors of type I by using a TGF-beta dependent promoter or a GFP-Smad2 fusion protein. This assay detects the activity far downstream in the TGF-beta receptor pathway, which cannot be allocated to an activity of a specific receptor.

Monitoring the activation of TGF-beta receptors directly could help to resolve the involvement of individual TGF-beta receptors and their ligands in specific signalling events.

The objective of the present invention is to provide a construct that allows detecting TGF-beta receptor activity directly, live and *in vivo* with subcellular resolution. A further objective of the present invention is to provide a method to test compounds for effects on TGF-beta receptor activation and to screen compound libraries for modulators of TGF-beta receptor activation.

### Summary of the invention:

To solve the first objective the present invention provides a fusion protein as reporter construct to detect TGF-beta receptor activation comprising
a. a type I TGF-beta receptor,
b. a circularly permutated fluorescent protein moiety (cpFP),
c. an activation state specific receptor binding domain, meaning a domain specifically binding to either the activated or inactive form of the TGF-beta receptor,
wherein the circularly permutated fluorescent protein moiety is inserted between the C-terminus of the TGF-beta receptor and the N-terminus of the activation state specific receptor binding domain (as defined in c.).

The term circularly permutated fluorescent protein moiety (cpFP) stands for a protein in which the N-terminus and C-terminus of a fluorescent protein (FP) are exchanged in order and coupled via a short linker. The circularly permutated fluorescent protein moiety can adopt two conformations. One conformation is further referred to as "free" conformation, as it is the conformation wherein the cpFP spontaneously folds into, when there are no spatial restrictions. The other conformation is further referred to as "restricted" conformation as it is the conformation the cpFP folds into, when the activation state specific receptor binding domain binds to the intracellular domain of the receptor forcing the cpFP, which is inserted between them, into a different conformation. The two different conformations have different degrees of fluorescence, including no fluorescence at all. The interaction of the N- and C-terminal fusion partners modulates these fluorescence levels.

According to the invention, an activation specific interaction between the receptor and the C-terminal activation state specific receptor binding domain therefore modulates the fluorescence of the cpFP inserted in between. In consequence, the activation of the receptor leads to a change in fluorescence of the cpFP. The change in fluorescence can be either an increase or decrease. This is due to the fact that, dependent on the protein sequence of the cpFP, either the restricted or the free conformation is fluorescent. In both conformations the overall beta barrel structure of the fluorescent protein is formed but becomes distorted by the interaction of the N- and C-terminal ends in the restricted conformation. Therefore the change from one conformation to the other does not involve a complete refolding and is consequently very fast. Preferably, the fusion protein is constructed in such a way that the intensity of the fluorescence of the circularly permutated fluorescent protein moiety increases upon activation of the receptor.

In the fusion protein according to the invention the fluorescent circularly permutated protein moiety is N-terminally linked to the C-terminus of a type I TGF-beta receptor and C-terminally linked to the N-Terminus of the activation state specific receptor binding domain.

The activation state specific receptor binding domain binds to an intracellular domain called GS domain. The GS domain is phosphorylated in the active receptor and nonphosphorylated in the inactive receptor.

In one embodiment the activation state specific receptor binding domain, preferably a FKBP12 moiety, binds exclusively to the inactive receptor, more precisely the non phosphorylated form of the GS domain. In this state the circularly permutated fluorescent protein (cpFP) is forced into a restricted conformation. Upon activation of the TGF-beta receptor, the GS domain becomes phosphorylated, and the activation state specific receptor binding domain dissociates from the receptor, which allows the circularly permutated fluorescent protein to fold back into the free conformation leading to a change in fluorescence.

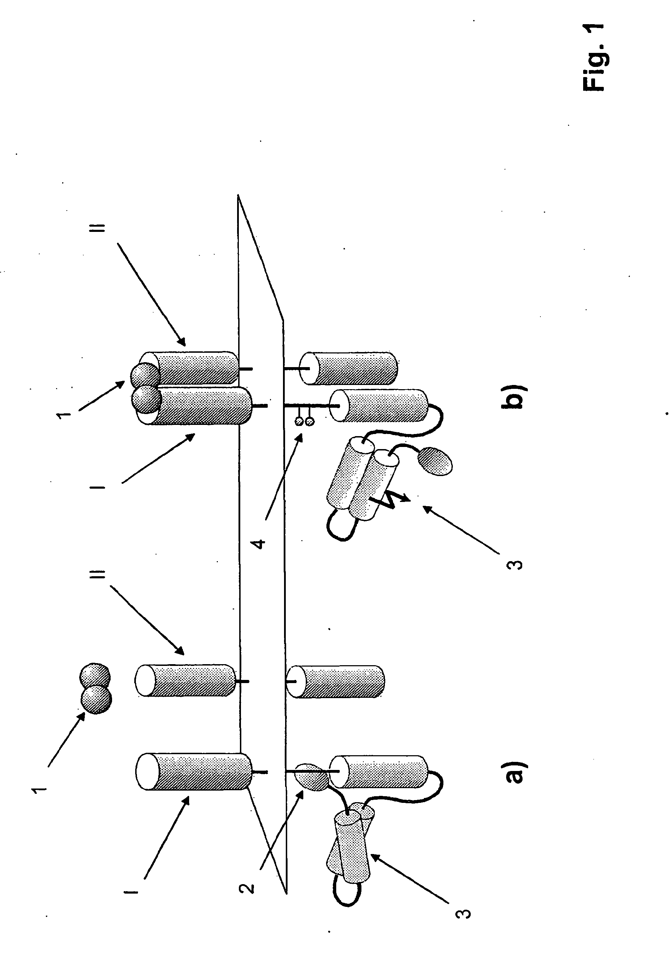

Advantageously, this insertion does not disrupt the functionality of the TGF-beta receptor: Since the FKBP12 (acting as inhibitor) is released after reporter activation just as during normal signalling, the phosphorylated binding site becomes available for recruiting the R-SMAD transcription factor that is the substrate of the type I receptor S/T kinase. Correspondingly, the inventors have verified that this reporter construct remains fully functional and that its ubiquitous expression in transgenic flies can complement the complete loss of the endogenous receptor. The principle of action of this fusion protein according to the invention is further illustrated in Fig. 1.

In an alternative embodiment the activation state specific receptor binding domain is a domain binding exclusively to the active receptor, more precisely the phosphorylated form of the GS domain. Upon activation of the TGF-beta receptor, the GS domain becomes phosphorylated and the circularly permutated fluorescent protein (cpFP) is forced from a free into a restricted conformation leading to a change in fluorescence. In this embodiment the activation state specific receptor binding domain is preferably a Mad Homology 2 (MH2) domain of an R-SMAD. As a consequence the reporter construct in this embodiment has a dominant negative effect on the signaling pathway measured, as the binding of the MH2 domain of the reporter construct to the activated receptor prevents recruitment and activation of the naturally occurring R-SMAD.

The invention thus provides a reporter construct that allows the direct detection of the activation of the receptor. Advantageously this activation can be followed directly in a living cell or organism using a standard fluorescence microscope, and allows the detection of the subcellular location of activated receptors in the cell. As the change in fluorescence signal occurs very fast, preferably in the range of seconds, upon activation, the activation can be measured almost in real time.

In contrast to other approaches the reporter construct according to the invention is not based on Förster-Resonance-Energie-Transfer (FRET) but uses instead only a single circularly, permutated fluorescent protein. Thus, receptor activation levels can be .measured directly by quantitative fluorescence microscopy without the technically challenging controls required with FRET-based systems. The reporter is therefore also useable for subcellular resolution imaging, single molecule fluorescence spectroscopy, and fluorescence recovery after photobleaching (FRAP) assays that can be used to monitor the dynamics of the activated receptors within the cell, as well as fluorescence activated cell sorting (FACS) which can be applied to distinguish and separate activated from non-activated cells.

The direct, fluorescence based detection of receptor activation is also superior to current methods for high content compound screening, as labour intensive and potentially error prone procedures such as enzyme linked immunoassays can be circumvented.

The term TGF-beta receptor according to the invention is membrane-spanning protein belonging to the TGF-beta receptor superfamily, with an extra-cellular domain binding a ligand, a trans-membrane domain and an intracellular serine/threonine kinase domain.

The TGF- beta receptor according to the invention preferably binds as ligand a protein belonging to the TGF-beta family of signalling molecules. Non limiting examples of the ligands include Activins and Inhibins, Anti-Müllerian hormone (AMH), Bone morphogenetic proteins (in particular BMP2 to BMP7), Decapentaplegic protein, Growth differentiation factors (in particular GDF1 to GDF15), Nodal and Lefty, TGF-beta and isoforms of these ligands, like TGF-β1, TGF-β2 and TGF-β3.

The fusion protein according to the invention contains a receptor of the TGF-beta receptor type I, which are also referred to as the Activin receptor-like Kinases (ALKs). Preferred examples include ACVR1B (ALK4), bone morphogenetic protein receptors (like BMPR1A (ALK3) and BMPR1B (ALK6)), TGFBR1 (ALK5) andACVR1C (ALK7), fly type I receptor BMP Thickveins (Tkv).

The TGF-beta receptor is preferably of one of the following sequences or a homologue thereof, with a sequence identity of over 60 %, preferably over 70 %, most preferably over 80%.

### SEQ ID No. 1 (Drosophila Tkv)

### SEQ ID No. 2 (Danio rerio ALK3)

### SEQ ID No. 3 (Homo sapiens ALK2)

### SEQ ID No. 4 (Homo sapiens ALK3)

### SEQ ID No. 5 (Homo sapiens ALK6)

### SEQ ID No. 6 (Homo sapiens ALK7)

In the circularly permutated fluorescent protein moiety (cpFP) the N-terminus and C-terminus of a fluorescent protein (FP) are exchanged in order and coupled via a short linker. The linker inside the cpFP is preferably between 2 and 20 amino acids long, preferably 4 to 6 amino acid residues, chosen from Glycine, Alanine and polar residues like Serine, Threonine, Glutamine or Asparagine, a particular preferred linker being GGSGG. The fluorescent protein (FP) is preferably an *Aequorea*-related fluorescent protein (GFP, EGFP, YFP, EYFP, CFP, T-Sapphire and other variants thereof) or a *Discosoma*-related fluorescent protein (RFP and other monomeric variants of DsRed).

The circularly permutated fluorescent protein moiety (cpFP) comprises preferably the following sequence in order from the N-terminus to the C-terminus:
(1) the C-terminus of the fluorescent protein (FP) or a variant thereof with a length of 105 to 115 amino acids residues,
(2) a linker sequence,
(3) the N-terminus of the fluorescent protein (FP) or a variant thereof with a length of 140 to 150 amino acids residues.

This especially holds true, when the cpFP is derived from an *Aequorea*-related fluorescent protein (FP).

In the cpFP the amino and carboxy termini of the FP are linked by the linker peptide and thus become internal amino acids. Consequently the amino and carboxy terminal ends of the cpFP are different from the amino-terminal and carboxy-terminal amino acids of the FP.

Preferably the FP is an *Aequorea*-related fluorescent protein moiety and the cpFP comprises: the amino-terminal end of the circularly permuted *Aequorea*-related fluorescent protein moiety is selected from the group consisting of E 142, Y 143, Y145, H148, D155, H169, E172, D173, A227 and 1229, and the carboxy-terminal end of the circularly permuted *Aequorea*-related fluorescent protein moiety is selected from the group consisting of N144, N146, N149, K162, K156, N170, I171, D 173, E172, A227, and I229.

The amino acid residue numbers mentioned above and below correspond to their location in the native *Aequorea* green fluorescent protein (SEQ ID No. 23) sequence.

Preferred variants of the FP are selected from the following mutations of an *Aeguorea*-related fluorescent protein moiety: V68L, Q69K, T203H, H148D T203F, H148T, T203F, H148D, T203F and F46L.

In certain embodiments the fusion protein comprises a circularly permutated fluorescent protein (cpFP) as described in EP1238982A1, WO0071565 or US7060793B2.

Whether the "free" or the "restricted" conformation of the cpFP is more fluorescent, depends on the sequence of the cpFP. In most cases the "restricted" conformation shows higher fluorescence, which as example holds true for the sequences according to SEQ ID No. 8 to 10. These, which are preferably used when the activation state specific receptor binding domain is a domain specifically binding to the active form of the TGF-beta receptor, lead to an increase in fluorescence upon receptor activation.

In a preferred embodiment, the cpFP carries the mutations H148T, T203F, as described by Nagai et al. (2001 PNAS 98: 3197-3202) as insertion between calmodulin and M13 and called "inverse-pericam". A modified and preferred sequence thereof is SEQ ID No. 7. This circular permutated fluorescent protein folds in its free conformation spontaneously into a fluorescent conformation. This cpFP is in particular preferred, when the activation state specific receptor binding domain is a domain specifically binding to the inactive form of the TGF-beta receptor, like FKBP12. In its restricted conformation, as long as the activation state specific receptor binding domain is bound to the receptor fluorescence is turned down or even off. Thus in this embodiment activation of the receptor will lead to an increase in fluorescence. In this embodiment the cpFP is preferably derived from EYFP, thus cpEYFP, and preferably also carrying the mutations V68L, Q69K and F46L.

Alternatively, especially in case the circularly permutated fluorescent protein moiety (cpFP) is derived from a *Discosoma*-related fluorescent protein the cpFP comprises preferably the following sequence in order from the N-terminus to the C-terminus:
(1) the C-terminus of the fluorescent protein (FP) or a variant thereof with a length of 205 to 215 amino acids residues,
(2) a linker sequence,
(3) the N-terminus of the fluorescent protein (FP) or a variant thereof with a length of 20 to 25 amino acids residues.
or alternatively:
(1) the C-terminus of the fluorescent protein (FP) or a variant thereof with a length of 55 to 60 amino acids residues,
(2) a linker sequence,
(3) the N-terminus of the fluorescent protein (FP) or a variant thereof with a length of 180 to 190 amino acids residues.

Preferably the circularly permutated fluorescent protein is selected from the group consisting of cpEYFP, cpEYFP(V68L/Q69K), cpEYFP(T203H), cpEYFP(V68L/Q69K/T203H), cpEYFP(H148D/T203F), cpEYFP(V68L/Q69K/H148D/T203F), cpEYFP(H148D/T203F/F46L), cpEYFP(V68L/Q69K/H148T/T203F/F46L)), cpEYFP(H148T/T203F), cpEYFP(V68L/Q69K/H148T/T203F) cpEYFP(H148T/T203F/F46L) and cpEYFP(V68L/Q69K/H148T/T203F/F46L).

The circular permutated fluorescent protein moiety is preferably of one of the following sequences or a homologue thereof, with a sequence identity of over 80 %, preferably over 90 %, most preferably over 95 %:
SEQ ID No. 7 (Inverse Pericam core)
SEQ ID No. 8 (ratiometric pericam core)
SEQ ID No. 9 (flash pericam core)
SEQ ID No. 10 (pericam core)

The activation state specific receptor binding domain, specifically binding to either the activated or inactive form of the TGF-beta receptor, binds preferably specifically to the intracellular GS-domain of the type I TGF-beta receptor in a activation-specific manner.

The activation state specific receptor binding domain binding specifically to the inactive form of the TGF-beta receptor is preferably the short protein called FK506-Binding Protein, 12-KD (FKBP12), also referred to as FK506- Binding Protein 1 (FKBP1), Proteinkinase C Inhibitor-2 (PKCI2) or PPIASE. The FKBP12 binds to the typeI receptor GS-domain exclusively when this domain is not phosphorylated.

The activation specific binding domain binding specifically to the inactive form of the TGF-beta receptor is preferably chosen out of one of the following sequences or a homologue thereof with a sequence identity of over 70 %, preferably over 75 %, most preferably over 85%:
SEQ ID No. 11 (Drosophila FKBP12)
SEQ ID NO. 12 (HUMAN FKBP1A)
SEQ ID NO. 13 (TRUNCATED HUMAN FKBP1A)

The FKBP12 in humans and other primates is most commonly called FKBP1 and has an additional N-terminal sequence that is not essential for the interaction with the GS domain. Thus for the purposes of the invention the human and other primate sequences are preferably truncated on the N-terminus in a way that they still comprise the domain of the FKPB_C superfamily. Preferably 37 amino acid residues are removed from the N-terminus of FKBP1. A preferred example of such a truncated sequence is SEQ ID No. 13.

The conservation between insect and zebrafish FKBP12 as well as human FKBP1A is sufficiently high so that reporter constructs comprising one of these proteins can be expected to work in the other organisms as well. This has been experimentally verified for the Drosophila FKBP12 that is functional as a reporter in combination with zebrafish ALK3 (SEQ. ID No.2) in zebrafish.

Alternatively, an activation state specific receptor binding domain binding specifically to the active form of the TGF-beta receptor can be derived from the MH2 domain of the receptor type specific R-SMADs, e.g. Smad 2/3 and their homologues for activin/TGF-beta receptor signalling and SMAD 1/5/8 and their homologues for BMP receptor signalling. These domains possess a basic patch specifically binding the phosphorylated form of the receptor GS domain (Wu et al., Science 287:92-97. Jan 2000) and can therefore be used to induce a closed conformation of the cpFP when the receptor is phosphorylated.

The activation specific binding domain binding specifically to the active form of the TGF-beta receptor is preferably chosen out of one of the following sequences or a homologue thereof with a sequence identity of over 70 %, preferably at least 75 %, most preferably at least 85 %:
SEQ ID NO. 14 Drosophila Mad MH2 domain
SEQ ID NO. 15 Drosophila Smox MH2 domain
SEQ ID NO. 16 Human Smad 1 MH2 domain
SEQ ID NO. 17 Human Smad 5 MH2 domain
SEQ ID NO. 18 Human Smad 2 MH2 domain
SEQ ID NO. 19 Human Smad 3 MH2 domain

In the fusion protein according to the invention the respective sequences of the TGF-beta receptor, the cpFP and the activation state specific receptor binding domain can be bound directly to each other or linked by a sequence of 1 to 20 amino acids, preferably 1 to 6 amino acids, preferably with amino acid residues chosen from Glycine, Alanine and polar residues like Serine, Threonine, Glutamine or Asparagine. Preferred linkers are selected from the single amino acid glycine, the dipeptides AG or TG and the tripeptides TGT, NGT, TGN, TAG, SAG or GAG, as well as the tetrapeptides NGTG, TGTG, GNGT, TGNG, GTAG, GSAG or GAGT.

The receptor protein and cpFP are preferably directly fused or linked by the tripeptide SAG.

In case that the activation state specific receptor binding domain is binding to the inactive receptor, like FKBP12, the cpFP and the activation state specific receptor binding domain are preferably directly fused or connected via an linker selected from the tripeptides TGT or NGT or the tetrapeptides TGTG or NGTG. In case the activation state specific receptor binding domain is binding to the active receptor, like for the MH2 domains, a longer linker, preferably with a length of 6 to 20 amino acids residues, is preferred.

Examples of the fusion protein according to the invention are given in the SEQ ID No. 20 and 21 and SEQ ID No. 24 to 28.

In another aspect, the invention provides a nucleic acid molecule, wherein the nucleic acid molecule encodes a fusion protein according to the invention. The term nucleic acid molecule does not only comprise DNA and RNA, but also nucleic acids with altered backbone, like for instance PNA. The nucleic acid molecule according to the invention comprises the coding sequence for a TGF-beta receptor including the intracellular GS domain, the coding sequence for a circularly permutated fluorescent protein in frame and downstream of the TGF-beta receptor, and the coding sequence for an activation state specific receptor binding domain of the TGF-beta receptor in frame and downstream of the circularly permutated fluorescent protein.

Further objects of the invention are an expression vector, comprising the nucleic acid molecule according to the invention, as well as a host cell or multi-cellular organism transformed with the nucleic acid molecule according to the invention. Humans are explicitly excluded from the term multi-cellular organisms in this context and in the following description.

The invention also comprises the use of an expression cassette or vector for the construction of a nucleic acid molecule encoding a reporter construct according to the invention. This expression cassette or vector comprises a cloning site for the insertion of a type I TGF-beta receptor coding sequence followed by a nucleic acid molecule encoding a circularly permutated fluorescent protein in frame and the coding sequence for the activation state specific receptor binding protein domain in frame and downstream of the coding sequence of the circularly permutated fluorescent protein. This expression cassette or vector advantageously allows the insertion of a nucleic acid encoding a type I TGF-beta receptor coding sequence of interest. In this way the person applying the invention can construct a reporter construct being specific for the type I TGF-beta receptor he is interested in, e. g. to study the activation of type I TGF-beta receptor or to detect compounds activating or modulating the activity of a type I TGF-beta receptor. An example of such an expression vector is given in the SEQ ID No. 22.

Another object of the invention is a host cell or multi-cellular organism transformed with the nucleic acid molecule according to the invention. As used herein, a "host cell" is a naturally occurring cell or a transformed cell or cell line that contains an expression vector and supports the replication or expression of an expression vector. Host cells may be cultured cells, explants, cells *in vivo,* and the like. Host cells are preferably eukaryotic cells such as insect, amphibian, or mammalian cells such as CHO, HeLa, HEK293 and the like.

A multi-cellular organism according to the invention is a naturally occurring or otherwise genetically modified organism, preferably an invertebrate or vertebrate organism, like *Drosophila melanogaster, Caenorhabditis elegans, Xenopus laevis,* Medaka or Zebrafish or *Mus musculus,* or an embryo thereof.

The invention comprises transient transfectants (e. g. by mRNA), plasmid transfectants as well as host cells or multi-cellular organisms, wherein the nucleic acid molecule according to the invention is stably integrated into the genome.

The invention further comprises a kit with at least one of the following components: a fusion protein, a nucleic acid molecule, an expression cassette or vector, a host cell or multicellular organism according to the invention and optionally control reagents, puffer or reagents for cell transfection.

The invention also comprises the use of a product according to the invention, in particular the fusion protein host cell or multicellular organism or the kit (as well as the nucleic acid or the expression cassette or vector) for monitoring the activation of TGF-beta receptor or to detect compounds activating or modulating the activity of a TGF-beta receptor.

Another object of the invention is a method for detecting TGF-beta receptor activation or detecting an effect a compound has on TGF-beta receptor activity, comprising the steps of:
a.) expressing the fusion protein according to one of the claims 1 to 7 in a host cell or multi-cellular organism; and
b.) measuring the fluorescence emitted by the fusion protein.

This method allows in one aspect to detect activation of the receptor by exogenous ligands or ligands present in the host cell or multi-cellular organism under specific conditions (e. g. a differentiation state, a specific cell type or cell culture conditions).

In another aspect the method is used for screening purposes and allows the screening of large compound libraries in a high content screening.

The screening can be in one aspect for compounds leading to an activation of the TGF-beta receptor, by performing the following steps:
a) adding the compound or compounds to a host cell or multi-cellular organism expressing the fusion according to the invention; and
b) measuring a fluorescence emitted by the fusion protein.

A change in fluorescence compared to the non-treated control (without adding the compound to be tested) indicates an agonist or inhibitor effect on activation of the receptor.

Preferably an increased fluorescence emitted by the fusion protein indicates receptor activation.

However, it is even more interesting to screen for compounds modulating TGF-beta receptor activity. In this embodiment of the screening method the compound or compounds to be tested are examined for an effect on the already activated TGF-beta receptor or its activation.

To detect modulators of TGF-beta receptor activity preferably a known activator of the TGF-beta receptor, like a TGF-beta growth factor, is added as additional compound preferably before or alternatively in parallel or after adding the compound to be tested.

Thus the TGF-beta receptor is preferably activated by this known activator. A change in fluorescence compared to the control treated only with the known activator (without adding the compound to be tested) indicates an modulating effect, e. g. an antagonist or partial antagonist effect on activation of the receptor.

This method to detect modulators of TGF-beta receptor activation comprises preferably the following steps:
a.) providing a known activator of the TGF-beta receptor, like a TGF-beta growth factor to a host cell expressing the fusion protein according to the invention and
b.) detecting a change in said fusion protein indicating receptor activation by detecting a signal from the fluorescent protein domain and
c.) adding a compound to be tested to the host cell and quantitatively observing changes in fluorescence intensity.

As the reporter protein according to the invention allows measuring receptor activation directly, it advantageously allows screening for compounds activating a specific TGF-beta receptor.

To detect compounds affecting signal transduction only through one or a specific group of TGF-beta receptors, the method is preferably performed with at least two different fusion proteins according to the invention. In this case the fusion proteins differ at least in their TGF-beta receptor component and optionally in the colour of the cpFP.

Performing the screen with reporter constructs containing different TGF-beta type I receptor allows the identification of receptor specific compounds affecting signal transduction only through one or a specific group of receptors. This is a major advantage of the invention and made possible due to the fact that the invention allows the direct detection of receptor activation. A screening for such receptor specific compounds is in principle not possible using assays for TGF-beta signal transduction known from the state of the art, as they operate further down in the signalling cascade where signalling from different receptors often converges.

This screening for receptor specific compounds can be either done in parallel assays using at least two different reporter constructs according to the invention containing different TGF-beta receptors or in one host cell or organism expressing at least two different reporter constructs with cpFPs of different colours.

The compounds to be tested in the screening method of the invention can be any small chemical compound, or a protein, sugar, nucleic acid or lipid. The assays of the invention can be designed to screen large chemical libraries by high throughput screening. In one preferred embodiment, high-throughput screening methods involve providing a compound library, including but not limited to, combinatorial chemical libraries, peptide or peptide mimetic or peptoidic libraries containing a large number of potential therapeutic compounds.

The invention is further illustrated by the following figures and examples without being limited to these:
**Fig. 1** illustrates the functional principle of the reporter construct according to the invention using an cpFP that is fluorescent in its free conformation and an activation state specific receptor binding domain, specifically binding to the inactive form of the TGF-beta receptor (like FKP12):
**Fig 1** **a.)** shows the reporter without ligand **1** bound to the surface. The activation state specific receptor binding domain **2** is bound to the intracellular GS-Domain. The circular permutated fluorescent protein (cpFP) **3** is turned into a non-fluorescent conformation. Fluorescence is OFF.
**Fig. 1 b)****:** Upon ligand binding **1**, the type II receptor (**II**) phosphorylates the GS domain of the type I receptor reporter construct(**I**). This displaces the activation state specific receptor binding domain **2,** allowing the cpFP **3** to adopt a fluorescent conformation. Fluorescence is ON.

The functionality of the fusion protein according to the invention is in the following examples illustrated by the example Drosophila Tkv reporter (further referred to as TIPF), a fusion protein consisting out of Drosophila Tkv, a TGF-beta type I receptor binding BMPs, an inverse pericam core and Drosophila FKBP12 linked by short linkers.

### Example 1: Construction of the Drosophila Tkv reporter (=TIPF) and mRNA injections into zebrafish:

The protein sequence of the *Drosophila* Tkv reporter (further referred to as TIPF) is listed below and in SEQ ID No. 20: Underlined: *Drosophila* Tkv
Bold : Inverse Pericam core
Italics: *Drosophila* FKBP12

This reporter for signalling via receptors for the BMP-subtype of TGF-beta ligands based on the Drosophila type I BMP receptor Thickveins (Tkv) was generated as follows: A fragment of the Tkv coding sequence (CDS) encompassing the C-terminal 62 aa, the CDS of fly FKBP12, and the Tkv 3'untranslated region (UTR) were amplified in separate PCR reactions from a custom cDNA library derived from 6-12h old Drosophila embryos. In parallel, the cpFP core domain was amplified from a plasmid containing the entire Inverse Pericam (Nagai et al., PNAS 98 (2001), 3197-3202, ). Using the respective external primers for fusion PCR, the Tkv C-terminus was joined to the cpFP core and FKBP12 to the Tkv 3'UTR. Both fusion pCR products were cloned into the pCR2.1 vector (Invitrogen) and joined using the shared KpnI site at the end of the cpFP core. The entire cassette was excised with EagI and XbaI and cloned into a pCS2 expression vector containing the Tkv cDNA, replacing the C-terminus and 3'UTR with the reporter fusion, Subsequently, the entire reporter construct was excised from the expression vector and transferred into the pUAST P-element transformation vector for the generation of transgenic flies expressing the reporter under control of the compound UAS/Gal4 expression system. In a final step, the UAS site was excised and replaced by the Drosophila Ubiquitin promoter for Gal4-independent ubiquitous expression.

The functionality of the fusion protein according to the invention was tested by mRNA injections into zebrafish (*Danio rerio*) embryos. It is known that fly BMP receptors like TKV, which belong to the transforming growth factor beta superfamily of proteins, can bind to and be activated by vertebrate BMPs (Holley et al., 1996). This approach is thus experimentally much faster than generating transgenic flies, which requires several generations of fly crossing per construct tested. TIPF mRNA was generated *in vitro* using the Message Machine kit (Ambion) using SP6 polymerase to transcribe the TIPF reporter cloned into the pCS2 expression vector.

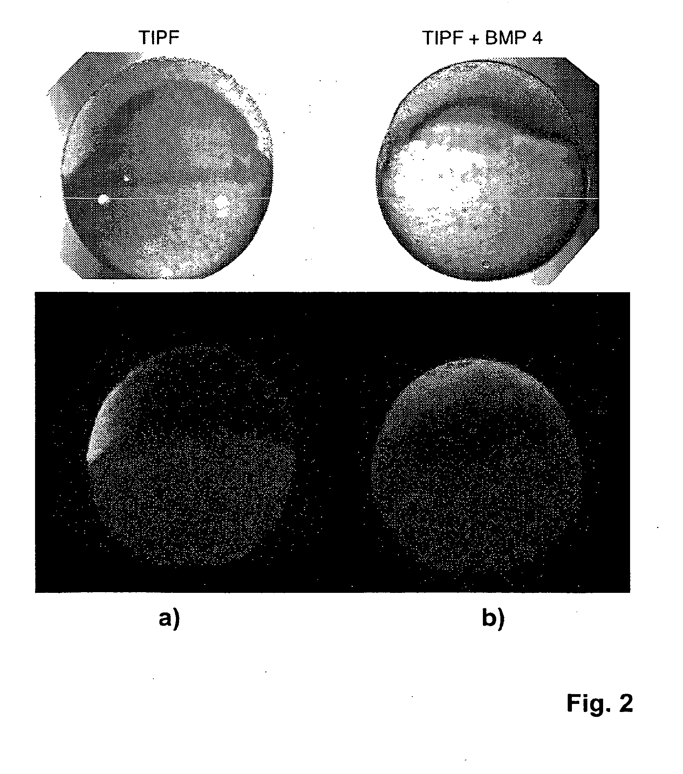

In the early fish embryo BMP signalling drives dorsoventral patterning. Ventral expression of several activating ligands (BMP 2, 4 and 7) and dorsal expression of BMP inhibitors (Chordin, Noggin) leads to a dorsoventral BMP activity gradient (Holley and Ferguson, 1997), where high signalling levels specify ventral and low levels dorsal fates. Although molecular markers can detect that asymmetry much earlier, the first morphologically detectable marker of dorsoventral patterning is the appearance of the shield on the dorsal side after about 6 hours of development, where the internalization of future mesoderm and endoderm starts. This stage is called shield stage. 50 to 100 pg of *in vitro* transcribed TIPF RNA was injected into zebrafish embryos at the one cell stage, and its fluorescence activity was observed at shield stage. Indeed, most embryos showed fluorescence restricted to the ventral side opposing the shield **(****Fig. 2a****)** where BMP signalling is known to be active. Coinjection of BMP4 mRNA (100pg/embryo) led to an expansion of the area of fluorescence **(****Fig. 2b****)** consistent with the observed ventralization of the embryo, i.e. the loss of dorsal fates that are normally specified at low levels of BMP signalling. These data suggest that the fusion protein according to the invention truthfully reflects localization of TGF-beta signalling activity.

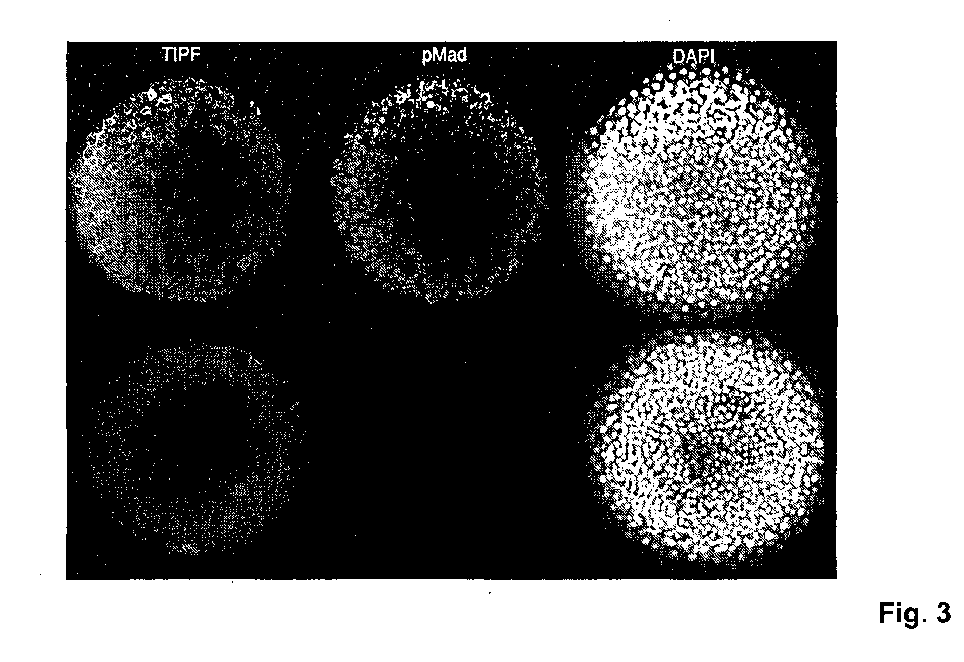

To confirm these results with molecular data, antibody stainings using a Rabbit and phospho-SMAD1/5/8 antibody (Cell Signalling Technology, 1:500) were performed on fixed *Danio rerio* embryos at a slightly younger stage of gastrulation (30% epiboly). While at that stage there is not yet a morphological marker for the prospective dorsal or ventral sides of the embryo, BMP pathway activation can be detected by accumulation of phosphorylated Smad1/5 transcription factors in the prospective ventral nuclei. Fluorescence of the TIPF reporter coincided with the region of BMP pathway activation as assayed by phospho-Smad immunostaining. As shown in **Fig. 3** (top), fluorescence of the TIPF reporter (left) coincides with the region of increased pSmad staining (centre). The right panel shows an overlay with nuclei staining using DAPI (4',6-Diamidino-2-phenylindol).

Consistently, coinjection of mRNA for the extracellular BMP inhibitor Chordin (100pg/embryo) both abolished pathway activity at the transcription factor level and suppressed TIPF fluorescence (Fig. 3, bottom, 30% epiboly, animal view). These data clearly show that the fusion protein according to the invention is working as a reporter for BMP receptor activation as predicted and reliably detects TGF-beta signalling activity at the receptor level.

### Example 2: Transgenic fly lines expressing the TIPF fusion protein

Using P-element mediated transgenesis with the plasmids described above transgenic fly lines were generated in a w¹¹¹⁸ background expressing the TIPF fusion protein under control of the ubiquitously expressed Ubiquitin promoter or in a tissue specific manner from the UAS promotor. For both transgenic construct insertions on all three major chromosomes were obtained. Insertions on the X chromosome were balanced with FM6 w⁻, 2^{nd} chromosome insertion were balanced with CyO and 3^{rd} chromosomal insertions with TM3, Sb.

The expected patterns of fluorescence could be detected e.g. in the wing disc (graded expression decaying with distance from the ligand source in the centre of the disc) in flies expressing the reporter either from the ubiquitously active ubiquitin promotor as well as from the UAS promoter in the presence of appropriate Gal4 drivers.

When expressed from the ubiquitin promoter, the reporter transgene is able to rescue the lethality associated with homozygous *tkv* mutants to full adult survival, demonstrating that the TIPF reporter remains a fully functional receptor.

### Example 3: A zebrafish ALK3 - based fusion protein according to the invention

A corresponding fusion protein was made by fusing the reporter cassette consisting of the Inverse Pericam core and Drosophila FKBP12 as described in example 1 to the zebrafish BMP receptor ALK3. The protein sequence of the zebrafish ALK3 reporter is listed below and in SEQ ID No. 21. Underlined:
Bold : Inverse Pericam core
Itaclics: *Drosophila* FKBP12

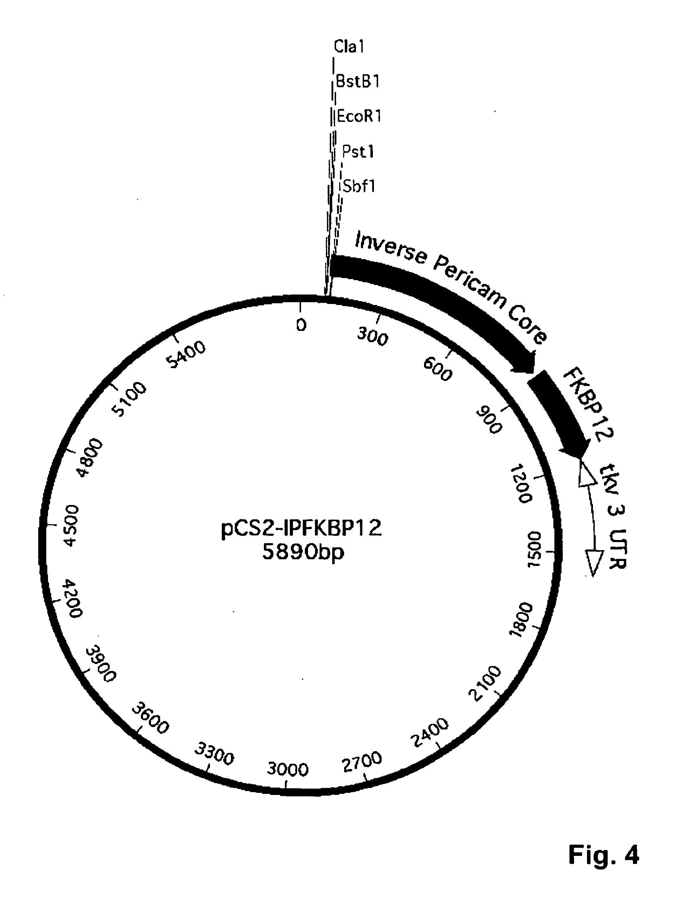

To generate the zebrafish ALK3 reporter construct, the Tkv CDS was excised from the TIPF reporter (as constructed in example 1) with PstI and EcoRI to obtain the linearized expression vector without the TGF-beta receptor according to SEQ ID No. 22. A plasmid map of this vector is given in **Fig. 4****.** In this vector the corresponding full length cDNA encoding the zebrafish ALK3 according to SEQ ID No. 2 was cloned, which was amplified from a custom cDNA library using primers carrying EcoRI (5'primer) and NsiI (3'primer).

Zebrafish assays were performed according to example 1. In all assays this reporter behaved like the fly version used in example 1, thus demonstrating the general applicability of the detection principle.

As verified for the zebrafish reporter and supported by the blast alignments below, conservation between insect and zebrafish as well as human FKBP12 is sufficiently high so that reporters comprising one of these proteins can be expected to work in the other organisms as well.

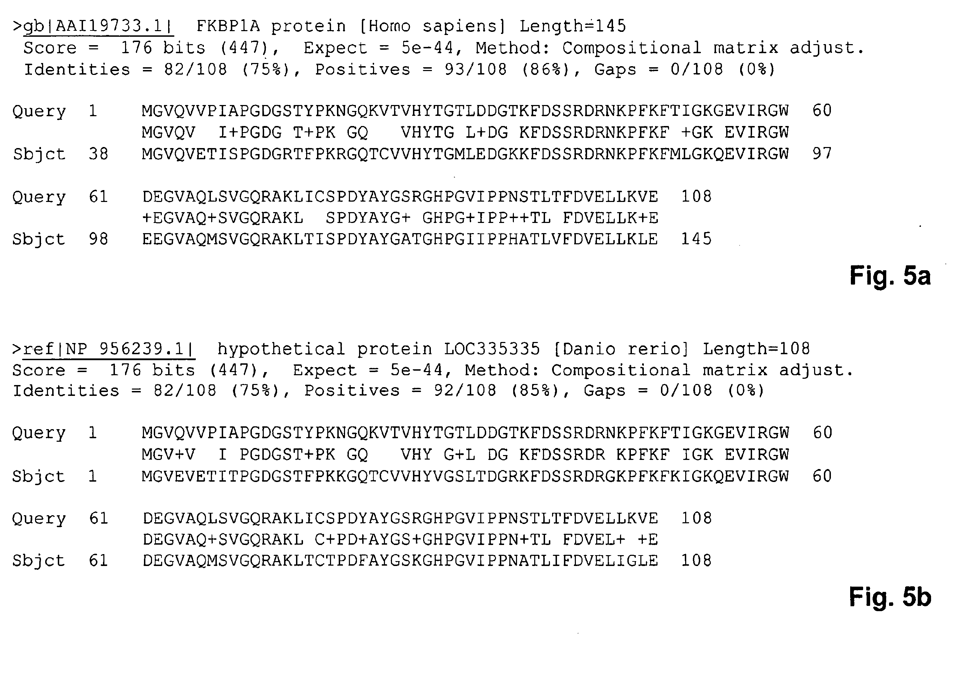

**Fig. 5a** and **Fig. 5b** show the alignments of drosophila FKBP12 (query) with the corresponding human (Fig. 5a) and zebrafish (Fig. 5b) sequences (Sbjct).

### Example 4: reporters for human ALKs

Corresponding reporters for the activation of human ALKs are made by cloning a human ALK reporter, preferably encoding a sequence according to one of the SEQ ID No. 1 to 6, into the vector according to SEQ ID No. 22.

The following examples are given for human ALK reporters: Underlined: *Homo sapiens* ALK2
Bold : Inverse Pericam core
Italics: *Drosophila* FKBP12 Underlined: *Homo sapiens* ALK3
Bold : Inverse Pericam core
Italics: *Drosophila* FKBP12 Underlined: *Homo sapiens* ALK6
Bold : Inverse Pericam core
Italics: *Drosophila* FKBP12 Underlined: *Homo sapiens* ALK7
Bold : Inverse Pericam core
Italics: *Drosophila* FKBP12

The Drosophila FKBP12 can but does not have to be replaced by a sequence encoding the truncated human FKBP according to SEQ ID No. 13. This is illustrated by showing a preferred protein sequence of the human ALK6 reporter listed below and in SEQ ID No. 28:

In the same way the FKBP12 can be replaced by one of the sequences according to SEQ ID No 14 to 19.

## Claims

1. A fusion protein comprising
a.) a type I TGF-beta receptor,
b.) a circularly permutated fluorescent protein moiety,
c.) an activation state specific receptor binding domain, specifically binding to either the activated or inactive form of the TGF-beta receptor,
wherein the circularly permutated fluorescent protein moiety is inserted between the C-terminus of the TGF-beta receptor and the N-terminus of the activation state specific
receptor binding domain as defined in c.).

2. A fusion protein according to claim 1 wherein the intensity of the fluorescence of the circularly permutated fluorescent protein moiety increases upon activation of the receptor.

3. A fusion protein according to claim 1 or 2 wherein the type I TGF-beta receptor is chosen from Activin receptor-like Kinases (ALKs) including ACVR1B (ALK4), bone morphogenetic protein receptors (like BMPR1A (ALK3) and BMPR1B (ALK6)), TGFBR1 (ALK5), ACVR1C (ALK7) and fly type I receptor BMP Thickveins (Tkv) and the sequences according to SEQ ID No. 1 to 7 or a homolog thereof with a sequence identity of over 60 %.

4. A fusion protein according to one of the claims 1 to 3 wherein the circularly permutated fluorescent protein is derived from an *Aequorea*-related fluorescent protein or a *Discosoma*-related fluorescent protein.

5. A fusion protein according to one of the claims 1 to 4 wherein the circularly permutated fluorescent protein moiety is selected from the group consisting of cpGFP and cpEYFP, as well as cpGFP or cpEYFP with one or several of the following mutations V68L, Q69K, T203H, H148D, T203F, H148T, T203F, H148D, T203F and F46L, or is chosen from a sequence according to SEQ ID No. 8 to 10 or a homolog thereof with a sequence identity of over 80 %.

6. A fusion protein according to one of the claims 1 to 5 wherein the activation state specific receptor binding domain is FKBP-12 or a Mad Homology 2 (MH2) domain of a R-SMAD, preferably chosen from a sequence according to SEQ ID No. 11 to 19 or a homolog thereof with a sequence identity of over 70 %.

7. A fusion protein according to one of the claims 1 to 5 with a sequence chosen from one of the sequences according to SEQ ID No. 20 and 21, as well as SEQ ID No. 24 to 28.

8. A nucleic acid molecule encoding a fusion protein according to one of the claims 1 to 7.

9. An expression cassette or vector comprising a nucleic acid molecule according to claim 8.

10. Use of an expression cassette or vector for the construction of an expression cassette or vector according to claim 9, wherein the expression cassette or vector used comprises a cloning site for the insertion of a type I TGF-beta receptor coding sequence followed by a nucleic acid molecule encoding a circularly permutated fluorescent protein in frame and the coding sequence for an activation state specific receptor binding protein domain as defined in claim 1 in frame and downstream of the coding sequence of the circularly permutated fluorescent protein.

11. A host cell or a non human multicellular organism containing a nucleic acid according to claim 8 or expression cassette or vector according to claim 9 and/or expressing a protein according to one of the claims 1 to 7.

12. A Kit comprising:
a.) a fusion protein according to one of the claims 1 to 7 and/or
b.) a nucleic acid molecule according to claim 8 and/or
c.) an expression cassette or vector according to claim 9 and/or
d.) a host cell or multicellular organism according to claim 11 and optionally control reagents, buffers or reagents for cell transfection.

13. Use of a product according to one of the claims 1 to 9, 11 or 12 for monitoring the activation of TGF-beta receptor or to detect compounds activating or modulating the activity of a TGF-beta receptor.

14. Method for detecting TGF-beta receptor activation or detecting an effect a compound has on TGF-beta receptor activity, comprising the steps of:
a.) expressing the fusion protein according to one of the claims 1 to 7 in a host cell or non-human multi-cellular organism ; and
b.) measuring the fluorescence emitted by the fusion protein

15. Method according to claim 14 used for detecting activation of the receptor by exogenous ligands or ligands present in the host cell or multi-cellular organism under specific conditions.

16. Method according to claim 14 used for detecting modulators of TGF-beta receptor activity comprising activating the TGF-beta receptor and adding a compound to be tested before, in parallel or after activating the TGF-beta receptor and measuring changes in fluorescence emitted by the fusion protein in response to the addition of the compound to be tested.

17. Method according to one of the claim 14 to 16 used to detect compounds affecting signal transduction only through one or a specific group of TGF-beta receptors, comprising performing the method with at least two different fusion proteins according to one of the claims 1 to 7, whereas the fusion proteins differ in their type I TGF-beta receptor component.

## Patentansprüche

1. Fusionsprotein enthaltend:
a.) ein Typ I-TGF-beta-Rezeptor,
b.) eine zirkulär permutierte fluoreszierende Proteineinheit,
c.) eine Aktivierungszustand-spezifische Rezeptor-Bindungsdomaine, welche entweder die aktivierte oder inaktive Form des TGF-beta-Rezeptors spezifisch bindet,
wobei die zirkulär permutierte fluoreszierende Proteineinheit zwischen dem C-Terminus des TGF-beta-Rezeptors und dem N-Terminus der wie in c.) definierten Aktivierungszustand-spezifischen Rezeptor-Bindungsdomain eingefügt ist.

2. Fusionsprotein nach Anspruch 1, wobei die Fluoreszenzintensität der zirkulär permutierten fluoreszierenden Proteineinheit nach Aktivierung des Rezeptors zunimmt.

3. Fusionsprotein nach Anspruch 1 oder 2, wobei der Typ I-TGF-beta-Rezeptor ausgewählt ist aus Activin-Receptor-like Kinases (ALKs) einschließlich ACVR1B (ALK4), Rezeptoren für knochenmorphogenetische Proteine (wie BMPR1A (ALK3) and BMPR1B (ALK6)), TGFBR1 (ALK5), ACVR1C (ALK7) und dem Fliegen-Typ-I-Rezeptor BMP Thickveins (Tkv) und den Sequenzen gemäß SEQ ID Nr. 1 bis 7 oder einem Homologen davon mit einer Sequenzidentität von über 60 %.

4. Fusionsprotein nach einem der Ansprüche 1 bis 3, wobei das zirkulär permutierte fluoreszierende Protein abgeleitet ist von einem *Aequorea*-verwandten fluoreszierenden Protein oder einem *Discosoma*- verwandten fluoreszierenden Protein.

5. Fusionsprotein nach einem der Ansprüche 1 bis 4, wobei das zirkulär permutierte fluoreszierende Protein ausgewählt ist aus der Gruppe bestehend aus cpGFP und cpEYFP, sowie cpGFP oder cpEYFP mit einer oder mehrerer der folgenden Mutationen V68L, Q69K, T203H, H148D, T203F, H148T, T203F, H148D, T203F und F46L, oder ausgewählt ist aus einer Sequenz gemäß SEQ ID Nr. 8 bis 10 oder einem Homologen davon mit einer Sequenzidentität von über 80 %.

6. Fusionsprotein nach einem der Ansprüche 1 bis 5, wobei die Aktivierungszustand-spezifische Rezeptor-Bindungsdomaine FKBP-12 oder eine Mad Homology 2 (MH2)-Domain eines R-SMADs ist, bevorzugt ausgewählt aus einer Sequenz gemäß SEQ ID Nr. 11 bis 19 oder Homologen davon mit einer Sequenzidentität von über 70 %.

7. Fusionsprotein nach einem der Ansprüche 1 bis 5, mit einer Sequenz ausgewählt aus den Sequenzen gemäß SEQ ID Nr. 20 und 21, sowie SEQ ID Nr. 24 bis 28.

8. Nukleinsäuremolekül, welches für ein Fusionsprotein gemäß einem der Ansprüche 1 bis 7 kodiert.

9. Expressionskassette oder Vektor enthaltend ein Nukleinsäuremolekül gemäß Anspruch 8.

10. Verwendung einer Expressionskassette oder eines Vektors für die Herstellung einer Expressionskassette oder eines Vektors gemäß Anspruch 9, wobei die verwendete Expressionskassette oder der verwendete Vektor eine Klonierungsstelle für das Einfügen einer für einen Typ I-TGF-beta-Rezeptor kodierenden Sequenz, gefolgt von einem Nukleinsäuremolekül kodierend für eine zirkulär permutierte fluoreszierende Proteineinheit wie in Anspruch 1 definiert und wiederum gefolgt von einer kodierenden Sequenz für die Aktivierungszustand-spezifische Rezeptor-Bindungsdomaine im selben Leseraster enthält

11. Wirtszelle oder nicht-menschlicher multizellulärer Organismus enthaltend eine Nukleinsäure gemäß Anspruch 8 oder eine Expressionskassette oder einen Velctor gemäß Anspruch 9 und/oder ein Protein gemäß einem der Ansprüche 1 bis 7 exprimierend.

12. Kit enthaltend:
a.) ein Fusionsprotein nach einem der Ansprüche 1 bis 7 und/oder
b.) ein Nukleinsäuremolekül nach Anspruch 8 und/oder
c.) eine Expressionskassette oder einen Velctor nach Anspruch 9 und/oder
d.) eine Wirtszelle oder ein multizellulärer Organismus nach Anspruch 11 und optional Kontrollreagenzien, Puffer oder Reagenzien für die Zelltransfektion.

13. Verwendung eines Erzeugnisses nach einem der Ansprüche 1 bis 9, 11 oder 12 für die Messung der Aktivierung eines TGF-beta-Rezeptors oder zur Detektion von Verbindungen, welche eines TGF-beta-Rezeptor aktivieren oder dessen Aktivität modulieren.

14. Verfahren zum Nachweis der TGF-beta-Reze-ptor-Aktivierung der zur Detektion eines Effekts, welchen eine Verbindung auf die TGF-beta-Rezeptor-Aktivität hat, welches folgende Schritte umfasst:
a.) Expression des Fusionsproteins gemäß einem der Ansprüche 1 bis 7 in einer Wirtszelle oder einem nicht-menschlichen multizellulären Organismus und
b.) Messen der Fluoreszenz, welche durch das Fusionsprotein emittiert wird.

15. Verfahren nach Anspruch 14, verwendet zum Nachweis der Aktivierung des Rezeptors durch exogene Liganden oder Liganden, welche in der Wirtszelle oder dem multizellulären Organismus unter spezifischen Bedingungen vorhanden sind.

16. Verfahren nach Anspruch 14, verwendet zur Detektion von Modulatoren der TGF-beta-Rezeptor-Alctivität umfassend das Aktivierten des TGF-beta-Rezeptors und das Zugeben einer Testverbindung vor, parallel oder nach dem Aktivierten des TGF-beta-Rezeptors und Messen der durch die Testsubstanz hervorgerufenen Änderung in der durch das Fusionsprotein emittierten Fluoreszenz.

17. Verfahren nach einem der Ansprüche 14 bis 16, verwendet zur Detektion von Substanzen, welche die Signaltransduktion nur durch einen oder eine spezifische Gruppe von TGF-beta-Rezeptoren beeinflussen, wobei das Verfahren mit mindestens zwei verschiedenen Fusionsproteinen nach einem der Ansprüche 1 bis 7 durchgeführt wird, die sich in der Typ I-TGF-beta-Rezeptor-Komponente unterscheiden.

## Revendications

1. Protéine de fusion comprenant :
a.) un récepteur TGF-bêta de typé I,
b.) une entité de protéine fluorescente circulairement permutée,
c.) un domaine de liaison à un récepteur spécifique d'état d'activation, se liant spécifiquement soit à une forme active, soit à une forme inactive du récepteur TGF-bêta,
dans laquelle le groupement de protéine fluorescente circulairement permutée est inséré entre la terminaison C du récepteur TGF-bêta et la terminaison N du domaine de liaison à un récepteur spécifique d'état d'activation tel que défini en c.).

2. Protéine de fusion selon la revendication 1, dans laquelle l'intensité de la fluorescence une entité de protéine fluorescente circulairement permutée augmente lors de l'activation du récepteur.

3. Protéine de fusion selon la revendication 1 ou 2, dans laquelle le récepteur TGF-bêta de type I est choisi groupe constitué de kinases de type récepteur d'activine (ALK) comprenant ACVR1B (ALK 4), des récepteurs pour des protéines morphogénétiques de l'os (comme BMPR1A) (ALK 3) et BMPR1B(ALK 6)), TGFBR1 (ALK 5), ACVR1C (ALK 7) et le récepteur Thickveins pour protéine morphogénétique de l'os (BMP) de type I de drosophile (Tkv) et les séquences selon le numéro d'identification de séquence 1 à 7 ou un homologue de ceux-ci ayant une identité de séquence supérieure à 60 %.

4. Protéine de fusion selon l'une des revendications 1 à 3, dans laquelle la protéine fluorescente circulairement permutée est obtenue à partir d'une protéine fluorescente associée à une méduse *Aequorea* ou d'une protéine fluorescente associée à un corail *Discosoma.*

5. Protéine de fusion selon l'une des revendications 1 à 4, dans laquelle le groupement de protéine fluorescente circulairement permutée est sélectionné groupe constitué de cpGFP et de cpEYFP, ainsi que de cpGFP ou de cpEYFP présentant une ou plusieurs des mutations suivantes V68L, Q69K, T203H, H148D, T203F, H148T, T203F, H148D, T203F et F46L, ou est choisi à partir d'une séquence selon le numéro d'identification de séquence 8 à 10 ou un homologue de ceux-ci présentant une identité de séquence supérieure à 80%.

6. Protéine de fusion selon l'une des revendications 1 à 5, dans laquelle le domaine de liaison à un récepteur spécifique d'état d'activation est FKBP-12 ou un domaine (MH2) d'homologie à Mad 2 d'une protéine R-SMAD, de préférence choisi à partir d'une séquence selon le numéro d'identification de séquence 11 à 19 ou d'un homologue de ceux-ci présentant une identité de séquence supérieure à 70 %.

7. Protéine de fusion selon l'une des revendications 1 à 5 présentant une séquence choisie à partir de l'une des séquences selon le numéro d'identification de séquence 20 et 21, de même que le numéro d'identification de séquence 24 à 28.

8. Molécule d'acide nucléique codant une protéine de fusion selon l'une des revendications 1 à 7.

9. Vecteur ou cassette d'expression comprenant une molécule d'acide nucléique selon la revendication 8.

10. Utilisation d'un vecteur ou d'une cassette d'expression pour la construction d'un vecteur ou d'une cassette d'expression selon la revendication 9, où le vecteur ou la cassette d'expression utilisé comprend un site de clonage pour l'insertion d'une séquence codant un récepteur TGF-bêta de type I suivie d'une molécule d'acide nucléique codant une protéine fluorescente circulairement permutée dans le cadre et de la séquence de codage pour un domaine de protéine de liaison à un récepteur spécifique d'état d'activation tel que défini selon la revendication 1 dans le cadre et en aval de la séquence de codage de la protéine fluorescente circulairement permutée.

11. Organisme multicellulaire non humain hôte ou cellule hôte contenant un acide nucléique selon la revendication 8 ou un vecteur ou une cassette d'expression selon la revendication 9 et/ou l'expression d'une protéine selon l'une des revendications 1 à 7.

12. Nécessaire comprenant :
a.) une protéine de fusion selon l'une des revendications 1 à 7 et/ou
b.) une molécule d'acide nucléique selon la revendication 8 et/ou
c.) un vecteur ou une cassette d'expression selon la revendication 9 et/ou
d.) un organisme multicellulaire hôte ou une cellule hôte selon la revendication 11 et optionnellement des réactifs de contrôle, des tampons ou des réactifs destinés à la transfection de cellule.

13. Utilisation d'un produit selon l'une des revendications 1 à 9, 11 ou 12 pour surveiller l'activation d'un récepteur TGF-bêta ou pour détecter des composants activant ou modulant l'activité d'un récepteur TGF-bêta.

14. Procédé de détection d'activation de récepteur TGF-bêta ou de détection d'un effet qu'un composant a sur l'activité de récepteur TGF-bêta, comprenant les étapes consistant à:
a.) exprimer la protéine de fusion selon l'une des revendications 1 à 7 dans un organisme multicellulaire non humain hôte ou une cellule hôte ; et
b.) mesurer la fluorescence émise par la protéine de fusion.

15. Procédé selon la revendication 14, utilisé pour détecter l'activation du récepteur par des ligands exogènes ou des ligands présents dans l'organisme multicellulaire hôte ou la cellule hôte dans des conditions spécifiques.

16. Procédé selon la revendication 14, utilisé pour détecter des modulateurs de l'activité de récepteur TGF-bêta comprenant l'activation du récepteur TGF-bêta et l'ajout d'un composant devant être testé avant, en parallèle ou après l'activation du récepteur TGF-bêta et pour mesurer les changements de fluorescence émise par la protéine de fusion en réponse à l'ajout du composant devant être testé.

17. Procédé selon l'une des revendications 14 à 16, utilisé pour détecter des composants ayant une influence sur la transduction de signal uniquement par le biais d'un groupement ou d'un groupement spécifique de récepteurs TGF-bêta, comprenant la mise en oeuvre du procédé avec au moins deux protéines de fusion différentes selon l'une des revendications 1 à 7, alors que les protéines de fusion diffèrent dans leur composant de récepteur TGF-bêta de type I.


### SEQUENCE LISTING

<110> Technische Universität Dresden
<120> A fluorescence based reporter construct for the direct detection of TGF-beta receptor activation and modulators thereof
<130> 17P0115EP
<160> 28
<170> PatentIn version 3.5
<210> 1
   <211> 509
   <212> PRT
   <213> Drosophila melanogaster
<400> 1
   Met Ala Pro Lys Ser Arg Lys Lys Lys Ala His Ala Arg Ser Leu Thr
   1               5                   10                  15
   Cys Tyr Cys Asp Gly Ser Cys Pro Asp Asn Val Ser Asn Gly Thr Cys
               20                  25                  30
   Glu Thr Arg Pro Gly Gly Ser Cys Phe Ser Ala Val Gln Gln Leu Tyr
           35                  40                  45
   Asp Glu Thr Thr Gly Met Tyr Glu Glu Glu Arg Thr Tyr Gly Cys Met
       50                  55                  60
   Pro Pro Glu Asp Asn Gly Gly Phe Leu Met Cys Lys Val Ala Ala Val
   65                  70                  75                  80
   Pro His Leu His Gly Lys Asn Ile Val Cys Cys Asp Lys Glu Asp Phe
                   85                  90                  95
   Cys Asn Arg Asp Leu Tyr Pro Thr Tyr Thr Pro Lys Leu Thr Thr Pro
               100                 105                 110
   Ala Pro Asp Leu Pro Val Ser Ser Glu Ser Leu His Thr Leu Ala Val
           115                 120                 125
   Phe Gly Ser Ile Ile Ile Ser Leu Ser Val Phe Met Leu Ile Val Ala
       130                 135                 140
   Ser Leu Cys Phe Thr Tyr Lys Arg Arg Glu Lys Leu Arg Lys Gln Pro
   145                 150                 155                 160
   Arg Leu Ile Asn Ser Met Cys Asn Ser Gln Leu Ser Pro Leu Ser Gln
                   165                 170                 175
   Leu Val Glu Gln Ser Ser Gly Ser Gly Ser Gly Leu Pro Leu Leu Val
               180                 185                 190
   Gln Arg Thr Ile Ala Lys Gln Ile Gln Met Val Arg Leu Val Gly Lys
   195 200 205
   Gly Arg Tyr Gly Glu Val Trp Leu Ala Lys Trp Arg Asp Glu Arg Val
   210 215 220
   Ala Val Lys Thr Phe Phe Thr Thr Glu Glu Ala Ser Trp Phe Arg Glu
   225 230 235 240
   Thr Glu Ile Tyr Gln Thr Val Leu Met Arg His Asp Asn Ile Leu Gly
   245 250 255
   Phe Ile Ala Ala Asp Ile Lys Gly Asn Gly Ser Trp Thr Gln Met Leu
   260 265 270
   Leu Ile Thr Asp Tyr His Glu Met Gly Ser Leu His Asp Tyr Leu Ser
   275 280 285
   Met Ser Val Ile Asn Pro Gln Lys Leu Gln Leu Leu Ala Phe Ser Leu
   290 295 300
   Ala Ser Gly Leu Ala His Leu His Asp Glu Ile Phe Gly Thr Pro Gly
   305 310 315 320
   Lys Pro Ala Ile Ala His Arg Asp Ile Lys Ser Lys Asn Ile Leu Val
   325 330 335
   Lys Arg Asn Gly Gln Cys Ala Ile Ala Asp Phe Gly Leu Ala Val Lys
   340 345 350
   Tyr Asn Ser Glu Leu Asp Val Ile His Ile Ala Gln Asn Pro Arg Val
   355 360 365
   Gly Thr Arg Arg Tyr Met Ala Pro Glu Val Leu Ser Gln Gln Leu Asp
   370 375 380
   Pro Lys Gln Phe Glu Glu Phe Lys Arg Ala Asp Met Tyr Ser Val Gly
   385 390 395 400
   Leu Val Leu Trp Glu Met Thr Arg Arg Cys Tyr Thr Pro Val Ser Gly
   405 410 415
   Thr Lys Thr Thr Thr Cys Glu Asp Tyr Ala Leu Pro Tyr His Asp Val
   420 425 430
   Val Pro Ser Asp Pro Thr Phe Glu Asp Met His Ala Val Val Cys Val
   435 440 445
   Lys Gly Phe Arg Pro Pro Ile Pro Ser Arg Trp Gln Glu Asp Asp Val
   450 455 460
   Leu Ala Thr Val Ser Lys Ile Met Gln Glu Cys Trp His Pro Asn Pro
   465 470 475 480
   Thr Val Arg Leu Thr Ala Leu Arg Val Lys Lys Thr Leu Gly Arg Leu
   485 490 495
   Glu Thr Asp Cys Leu Ile Asp Val Pro Ile Lys Ile Val
   500 505
<210> 2
   <211> 528
   <212> PRT
   <213> Danio rerio
<400> 2
   Met Arg Gln Leu Leu Phe Ile Thr Val Val Leu Thr Gly Val Cys Leu
   1               5                   10                  15
   Leu Leu Thr Leu Cys Ser Gly Ala Gly Gln Asn Pro Asp His Val Leu
               20                  25                  30
   Gln Gly Thr Gly Val Lys Leu Asp Ser Arg Arg Pro Gly Asp Asp Ser
           35                  40                  45
   Thr Ile Ala Pro Glu Asp Ala Ala Arg Phe Leu Ser Cys His Cys Ser
       50                  55                  60
   Gly His Cys Pro Asp Asp Ala Lys Asn Asn Thr Cys Glu Thr Asn Gly
   65                  70                  75                  80
   Gln Cys Phe Ala Ile Asn Glu Glu Asp Glu Asn Gly Asp Val Ile Leu
                   85                  90                  95
   Ser Ser Gly Cys Met Lys Tyr Glu Gly Ser His Phe Gln Cys Lys Asp
               100                 105                 110
   Ser Gln Phe Ala Gln Thr Arg Arg Thr Ile Glu Cys Cys Gln Phe Asp
           115                 120                 125
   Phe Cys Asn Gln Asp Leu Lys Pro Glu Leu Pro Pro Arg Asp Ser Glu
       130                 135                 140
   Pro Pro Asp Pro His Trp Leu Ala Phe Leu Ile Ser Val Thr Val Cys
   145                 150                 155                 160
   Phe Cys Ala Leu Ile Cys Val Thr Val Ile Cys Tyr Tyr Arg Tyr Lys
                   165                 170                 175
   Trp Gln Thr Glu Arg Gln Arg Tyr His Arg Asp Leu Glu Gln Asp Glu
               180                 185                 190
   Ala Phe Ile Pro Ala Gly Glu Ser Leu Lys Asp Leu Ile Asn Gln Ser
           195                 200                 205
   Gln Thr Ser Gly Ser Gly Ser Gly Leu Pro Leu Leu Val Gln Arg Thr
       210                 215                 220
   Ile Arg Lys Gln Ile Gln Thr Val Arg Met Ile Gly Lys Gly Arg Tyr
   225                 230                 235                 240
   Gly Glu Val Trp Leu Gly Arg Trp Arg Gly Glu Lys Val Ala Val Lys
                   245                 250                 255
   Val Phe Phe Thr Arg Glu Glu Ala Ser Trp Phe Arg Glu Thr Glu Ile
               260                 265                 270
   Tyr Gln Thr Val Leu Met Arg His Glu Asn Ile Leu Gly Phe Ile Ala
           275                 280                 285
   Ala Asp Ile Asn Gly Thr Gly Ala Ser Thr Gln Leu Tyr Leu Ile Thr
       290                 295                 300
   Asp Tyr His Glu Asn Gly Ser Leu Tyr Asp Tyr Leu Lys Phe Thr Thr
   305                 310                 315                 320
   Leu Asp Thr Gln Ala Leu Leu Arg Leu Ala Phe Ser Ala Ala Cys Gly
                   325                 330                 335
   Leu Cys His Leu His Thr Glu Ile Tyr Gly Thr Gln Gly Lys Pro Ala
               340                 345                 350
   Ile Ala His Arg Asp Leu Lys Ser Lys Asn Ile Leu Ile Lys Lys Asn
           355                 360                 365
   Gly Thr Cys Cys Ile Ala Asp Leu Gly Leu Ala Val Lys Phe Asn Ser
       370                 375                 380
   Asp Thr Asn Glu Val Asp Leu Pro Leu Ser Thr Arg Met Gly Thr Arg
   385                 390                 395                 400
   Arg Tyr Met Ala Pro Glu Val Leu Asp Glu Thr Leu Asn Lys Asn His
                   405                 410                 415
   Phe Gln Ala Tyr Ile Met Ala Asp Ile Tyr Ser Tyr Gly Leu Val Ile
               420                 425                 430
   Trp Glu Met Ala Arg Arg Cys Val Thr Gly Gly Ile Val Glu Glu Tyr
           435                 440                 445
   His Val Pro Tyr Tyr Glu Met Val Pro Ser Asp Pro Ser Tyr Glu Asp
       450                 455                 460
   Met Leu Glu Val Val Cys Val Lys Gly Leu Arg Pro Thr Val Ser Asn
   465                 470                 475                 480
   Arg Trp Asn Ser Asp Glu Cys Leu Arg Ala Met Leu Lys Leu Met Ser
   485 490 495
   Glu Cys Trp Ala His Asn Pro Ala Ser Arg Leu Thr Ile Leu Arg Val
   500 505 510
   Lys Lys Thr Leu Ala Lys Met Val Glu Ser Gln Asp Ile Lys Ile Tyr
   515 520 525
<210> 3
   <211> 509
   <212> PRT
   <213> Homo sapiens
<400> 3
<210> 4
   <211> 532
   <212> PRT
   <213> Homo sapiens
<400> 4
<210> 5
   <211> 502
   <212> PRT
   <213> Homo sapiens
<400> 5
<210> 6
   <211> 493
   <212> PRT
   <213> Homo sapiens
<400> 6
   Met Thr Arg Ala Leu Cys Ser Ala Leu Arg Gln Ala Leu Leu Leu Leu
   1               5                   10                  15
   Ala Ala Ala Ala Glu Leu Ser Pro Gly Leu Lys Cys Val Cys Leu Leu
               20                  25                  30
   Cys Asp Ser Ser Asn Phe Thr Cys Gln Thr Glu Gly Ala Cys Trp Ala
           35                  40                  45
   Ser Val Met Leu Thr Asn Gly Lys Glu Gln Val Ile Lys Ser Cys Val
       50                  55                  60
   Ser Leu Pro Glu Leu Asn Ala Gln Val Phe Cys His Ser Ser Asn Asn
   65                  70                  75                  80
   Val Thr Lys Thr Glu Cys Cys Phe Thr Asp Phe Cys Asn Asn Ile Thr
                   85                  90                  95
   Leu His Leu Pro Thr Ala Ser Pro Asn Ala Pro Lys Leu Gly Pro Met
               100                 105                 110
   Glu Leu Ala Ile Ile Ile Thr Val Pro Val Cys Leu Leu Ser Ile Ala
           115                 120                 125
   Ala Met Leu Thr Val Trp Ala Cys Gln Gly Arg Gln Cys Ser Tyr Arg
       130                 135                 140
   Lys Lys Lys Arg Pro Asn Val Glu Glu Pro Leu Ser Glu Cys Asn Leu
   145                 150                 155                 160
   Val Asn Ala Gly Lys Thr Leu Lys Asp Leu Ile Tyr Asp Val Thr Ala
                   165                 170                 175
   Ser Gly Ser Gly Ser Gly Leu Pro Leu Leu Val Gln Arg Thr Ile Ala
               180                 185                 190
   Arg Thr Ile Val Leu Gln Glu Ile Val Gly Lys Gly Arg Phe Gly Glu
           195                 200                 205
   Val Trp His Gly Arg Trp Cys Gly Glu Asp Val Ala Val Lys Ile Phe
   210 215 220
   Ser Ser Arg Asp Glu Arg Ser Trp Phe Arg Glu Ala Glu Ile Tyr Gln
   225 230 235 240
   Thr Val Met Leu Arg His Glu Asn Ile Leu Gly Phe Ile Ala Ala Asp
   245 250 255
   Asn Lys Asp Asn Gly Thr Trp Thr Gln Leu Trp Leu Val Ser Glu Tyr
   260 265 270
   His Glu Gln Gly Ser Leu Tyr Asp Tyr Leu Asn Arg Asn Ile Val Thr
   275 280 285
   Val Ala Gly Met Ile Lys Leu Ala Leu Ser Ile Ala Ser Gly Leu Ala
   290 295 300
   His Leu His Met Glu Ile Val Gly Thr Gln Gly Lys Pro Ala Ile Ala
   305 310 315 320
   His Arg Asp Ile Lys Ser Lys Asn Ile Leu Val Lys Lys Cys Glu Thr
   325 330 335
   Cys Ala Ile Ala Asp Leu Gly Leu Ala Val Lys His Asp Ser Ile Leu
   340 345 350
   Asn Thr Ile Asp Ile Pro Gln Asn Pro Lys Val Gly Thr Lys Arg Tyr
   355 360 365
   Met Ala Pro Glu Met Leu Asp Asp Thr Met Asn Val Asn Ile Phe Glu
   370 375 380
   Ser Phe Lys Arg Ala Asp Ile Tyr Ser Val Gly Leu Val Tyr Trp Glu
   385 390 395 400
   Ile Ala Arg Arg Cys Ser Val Gly Gly Ile Val Glu Glu Tyr Gln Leu
   405 410 415
   Pro Tyr Tyr Asp Met Val Pro Ser Asp Pro Ser Ile Glu Glu Met Arg
   420 425 430
   Lys Val Val Cys Asp Gln Lys Phe Arg Pro Ser Ile Pro Asn Gln Trp
   435 440 445
   Gln Ser Cys Glu Ala Leu Arg Val Met Gly Arg Ile Met Arg Glu Cys
   450 455 460
   Trp Tyr Ala Asn Gly Ala Ala Arg Leu Thr Ala Leu Arg Ile Lys Lys
   465 470 475 480
   Thr Ile Ser Gln Leu Cys Val Lys Glu Asp Cys Lys Ala
   485 490
<210> 7
   <211> 246
   <212> PRT
   <213> Artificial sequence
<220>
   <223> Inverse Pericam core
<400> 7
   Tyr Asn Ser Thr Asn Val Tyr Ile Met Ala Asp Lys Gln Lys Asn Gly
   1               5                   10                  15
   Ile Lys Ala Asn Phe Lys Ile Arg His Asn Ile Glu Asp Gly Gly Val
               20                  25                  30
   Gln Leu Ala Asp His Tyr Gln Gln Asn Thr Pro Ile Gly Asp Gly Pro
           35                  40                  45
   Val Leu Leu Pro Asp Asn His Tyr Leu Ser Phe Gln Ser Ala Leu Ser
       50                  55                  60
   Lys Asp Pro Asn Glu Lys Arg Asp His Met Val Leu Leu Glu Phe Val
   65                  70                  75                  80
   Thr Ala Ala Gly Ile Thr Leu Gly Met Asp Glu Leu Tyr Lys Val Asp
                   85                  90                  95
   Gly Gly Ser Gly Gly Thr Gly Val Ser Lys Gly Glu Glu Leu Phe Thr
               100                 105                 110
   Gly Val Val Pro Ile Leu Val Glu Leu Asp Gly Asp Val Asn Gly His
           115                 120                 125
   Lys Phe Ser Val Ser Gly Glu Gly Glu Gly Asp Ala Thr Tyr Gly Lys
       130                 135                 140
   Leu Thr Leu Lys Leu Ile Cys Thr Thr Gly Lys Leu Pro Val Pro Trp
   145                 150                 155                 160
   Pro Thr Leu Val Thr Thr Phe Gly Tyr Gly Leu Lys Cys Phe Ala Arg
                   165                 170                 175
   Tyr Pro Asp His Met Lys Gln His Asp Phe Phe Lys Ser Ala Met Pro
               180                 185                 190
   Glu Gly Tyr Val Gln Glu Arg Thr Ile Phe Phe Lys Asp Asp Gly Asn
           195                 200                 205
   Tyr Lys Thr Arg Ala Glu Val Lys Phe Glu Gly Asp Thr Leu Val Asn
       210                 215                 220
   Arg Ile Glu Leu Lys Gly Ile Asp Phe Lys Glu Asp Gly Asn Ile Leu
   225                 230                 235                 240
   Gly His Lys Leu Glu Tyr
                   245
<210> 8
   <211> 246
   <212> PRT
   <213> Artificial Sequence
<220>
   <223> Ratiometric Pericam core
<400> 8
   Tyr Asn Ser Asp Asn Val Tyr Ile Met Ala Asp Lys Gln Lys Asn Gly
   1               5                   10                  15
   Ile Lys Ala Asn Phe Lys Ile Arg His Asn Ile Glu Asp Gly Gly Val
               20                  25                  30
   Gln Leu Ala Asp His Tyr Gln Gln Asn Thr Pro Ile Gly Asp Gly Pro
           35                  40                  45
   Val Leu Leu Pro Asp Asn His Tyr Leu Ser Phe Gln Ser Ala Leu Ser
       50                  55                  60
   Lys Asp Pro Asn Glu Lys Arg Asp His Met Val Leu Leu Glu Phe Val
   65                  70                  75                  80
   Thr Ala Ala Gly Ile Thr Leu Gly Met Asp Glu Leu Tyr Lys Val Asp
                   85                  90                  95
   Gly Gly Ser Gly Gly Thr Gly Val Ser Lys Gly Glu Glu Leu Phe Thr
               100                 105                 110
   Gly Val Val Pro Ile Leu Val Glu Leu Asp Gly Asp Val Asn Gly His
           115                 120                 125
   Lys Phe Ser Val Ser Gly Glu Gly Glu Gly Asp Ala Thr Tyr Gly Lys
       130                 135                 140
   Leu Thr Leu Lys Leu Ile Cys Thr Thr Gly Lys Leu Pro Val Pro Trp
   145                 150                 155                 160
   Pro Thr Leu Val Thr Thr Phe Gly Tyr Gly Leu Lys Cys Phe Ala Arg
                   165                 170                 175
   Tyr Pro Asp His Met Lys Gln His Asp Phe Phe Lys Ser Ala Met Pro
               180                 185                 190
   Glu Gly Tyr Val Gln Glu Arg Thr Ile Phe Phe Lys Asp Asp Gly Asn
           195                 200                 205
   Tyr Lys Thr Arg Ala Glu Val Lys Phe Glu Gly Asp Thr Leu Val Asn
       210                 215                 220
   Arg Ile Glu Leu Lys Gly Ile Asp Phe Lys Glu Asp Gly Asn Ile Leu
   225                 230                 235                 240
   Gly His Lys Leu Glu Tyr
                   245
<210> 9
   <211> 246
   <212> PRT
   <213> Artificial Sequence
<220>
   <223> Flash Pericam core
<400> 9
   Tyr Asn Ser His Asn Val Tyr Ile Met Ala Asp Lys Gln Lys Asn Gly
   1               5                   10                  15
   Ile Lys Ala Asn Phe Lys Ile Arg His Asn Ile Glu Asp Gly Gly Val
               20                  25                  30
   Gln Leu Ala Asp His Tyr Gln Gln Asn Thr Pro Ile Gly Asp Gly Pro
           35                  40                  45
   Val Leu Leu Pro Asp Asn His Tyr Leu Ser His Gln Ser Ala Leu Ser
       50                  55                  60
   Lys Asp Pro Asn Glu Lys Arg Asp His Met Val Leu Leu Glu Phe Val
   65                  70                  75                  80
   Thr Ala Ala Gly Ile Thr Leu Gly Met Asp Glu Leu Tyr Lys Val Asp
                   85                  90                  95
   Gly Gly Ser Gly Gly Thr Gly Val Ser Lys Gly Glu Glu Leu Phe Thr
               100                 105                 110
   Gly Val Val Pro Ile Leu Val Glu Leu Asp Gly Asp Val Asn Gly His
           115                 120                 125
   Lys Phe Ser Val Ser Gly Glu Gly Glu Gly Asp Ala Thr Tyr Gly Lys
       130                 135                 140
   Leu Thr Leu Lys Leu Ile Cys Thr Thr Gly Lys Leu Pro Val Pro Trp
   145                 150                 155                 160
   Pro Thr Leu Val Thr Thr Phe Gly Tyr Gly Leu Lys Cys Phe Ala Arg
                   165                 170                 175
   Tyr Pro Asp His Met Lys Gln His Asp Phe Phe Lys Ser Ala Met Pro
               180                 185                 190
   Glu Gly Tyr Val Gln Glu Arg Thr Ile Phe Phe Lys Asp Asp Gly Asn
           195                 200                 205
   Tyr Lys Thr Arg Ala Glu Val Lys Phe Glu Gly Asp Thr Leu Val Asn
       210                 215                 220
   Arg Ile Glu Leu Lys Gly Ile Asp Phe Lys Glu Asp Gly Asn Ile Leu
   225                 230                 235                 240
   Gly His Lys Leu Glu Tyr
                   245
<210> 10
   <211> 246
   <212> PRT
   <213> Artificial Sequence
<220>
   <223> Pericam core
<400> 10
   Tyr Asn Ser His Asn Val Tyr Ile Met Ala Asp Lys Gln Lys Asn Gly
   1               5                   10                  15
   Ile Lys Ala Asn Phe Lys Ile Arg His Asn Ile Glu Asp Gly Gly Val
               20                  25                  30
   Gln Leu Ala Asp His Tyr Gln Gln Asn Thr Pro Ile Gly Asp Gly Pro
           35                  40                  45
   Val Leu Leu Pro Asp Asn His Tyr Leu Ser Tyr Gln Ser Ala Leu Ser
       50                  55                  60
   Lys Asp Pro Asn Glu Lys Arg Asp His Met Val Leu Leu Glu Phe Val
   65                  70                  75                  80
   Thr Ala Ala Gly Ile Thr Leu Gly Met Asp Glu Leu Tyr Lys Val Asp
                   85                  90                  95
   Gly Gly Ser Gly Gly Thr Gly Val Ser Lys Gly Glu Glu Leu Phe Thr
               100                 105                 110
   Gly Val Val Pro Ile Leu Val Glu Leu Asp Gly Asp Val Asn Gly His
           115                 120                 125
   Lys Phe Ser Val Ser Gly Glu Gly Glu Gly Asp Ala Thr Tyr Gly Lys
       130                 135                 140
   Leu Thr Leu Lys Leu Ile Cys Thr Thr Gly Lys Leu Pro Val Pro Trp
   145                 150                 155                 160
   Pro Thr Leu Val Thr Thr Phe Gly Tyr Gly Leu Lys Cys Phe Ala Arg
   165 170 175
   Tyr Pro Asp His Met Lys Gln His Asp Phe Phe Lys Ser Ala Met Pro
   180 185 190
   Glu Gly Tyr Val Gln Glu Arg Thr Ile Phe Phe Lys Asp Asp Gly Asn
   195 200 205
   Tyr Lys Thr Arg Ala Glu Val Lys Phe Glu Gly Asp Thr Leu Val Asn
   210 215 220
   Arg Ile Glu Leu Lys Gly Ile Asp Phe Lys Glu Asp Gly Asn Ile Leu
   225 230 235 240
   Gly His Lys Leu Glu Tyr
   245
<210> 11
   <211> 108
   <212> PRT
   <213> Drosophila melanogaster
<400> 11 '
<210> 12
   <211> 145
   <212> PRT
   <213> Homo sapiens
   <400> 12
   Met Gly Arg Gln Arg Ala Glu Gly Leu Gly Arg Ala Val Glu Pro Pro
   1               5                   10                  15
   Pro Gly Arg Cys Trp Ser Thr Pro Pro Val Ala Pro Pro Ala Arg Ser
               20                  25                  30
   Ala Ser Ala Ala Ala Met Gly Val Gln Val Glu Thr Ile Ser Pro Gly
           35                  40                  45
   Asp Gly Arg Thr Phe Pro Lys Arg Gly Gln Thr Cys Val Val His Tyr
       50                  55                  60
   Thr Gly Met Leu Glu Asp Gly Lys Lys Phe Asp Ser Ser Arg Asp Arg
   65                  70                  75                  80
   Asn Lys Pro Phe Lys Phe Met Leu Gly Lys Gln Glu Val Ile Arg Gly
                   85                  90                  95
   Trp Glu Glu Gly Val Ala Gln Met Ser Val Gly Gln Arg Ala Lys Leu
               100                 105                 110
   Thr Ile Ser Pro Asp Tyr Ala Tyr Gly Ala Thr Gly His Pro Gly Ile
           115                 120                 125
   Ile Pro Pro His Ala Thr Leu Val Phe Asp Val Glu Leu Leu Lys Leu
       130                 135                 140
   Glu
   145
<210> 13
   <211> 108
   <212> PRT
   <213> Homo sapiens
<400> 13
   Met Gly Val Gln Val Glu Thr Ile Ser Pro Gly Asp Gly Arg Thr Phe
   1               5                   10                  15
   Pro Lys Arg Gly Gln Thr Cys Val Val His Tyr Thr Gly Met Leu Glu
               20                  25                  30
   Asp Gly Lys Lys Phe Asp Ser Ser Arg Asp Arg Asn Lys Pro Phe Lys
           35                  40                  45
   Phe Met Leu Gly Lys Gln Glu Val Ile Arg Gly Trp Glu Glu Gly Val
       50                  55                  60
   Ala Gln Met Ser Val Gly Gln Arg Ala Lys Leu Thr Ile Ser Pro Asp
   65                  70                  75                  80
   Tyr Ala Tyr Gly Ala Thr Gly His Pro Gly Ile Ile Pro Pro His Ala
                   85                  90                  95
   Thr Leu Val Phe Asp Val Glu Leu Leu Lys Leu Glu
               100                 105
<210> 14
   <211> 179
   <212> PRT
   <213> Drosophila melanogaster
<400> 14
   Tyr Ser Glu Pro Ala Phe Trp Ala Ser Ile Ala Tyr Tyr Glu Leu Asn
   1               5                   10                  15
   Cys Arg Val Gly Glu Val Phe His Cys Asn Asn Asn Ser Val Ile Val
               20                  25                  30
   Asp Gly Phe Thr Asn Pro Ser Asn Asn Ser Asp Arg Cys Cys Leu Gly
           35                  40                  45
   Gln Leu Ser Asn Val Asn Arg Asn Ser Thr Ile Glu Asn Thr Arg Arg
       50                  55                  60
   His Ile Gly Lys Gly Val His Leu Tyr Tyr Val Thr Gly Glu Val Tyr
   65                  70                  75                  80
   Ala Glu Cys Leu Ser Asp Ser Ala Ile Phe Val Gln Ser Arg Asn Cys
                   85                  90                  95
   Asn Tyr His His Gly Phe His Pro Ser Thr Val Cys Lys Ile Pro Pro
               100                 105                 110
   Gly Cys Ser Leu Lys Ile Phe Asn Asn Gln Glu Phe Ala Gln Leu Leu
           115                 120                 125
   Ser Gln Ser Val Asn Asn Gly Phe Glu Ala Val Tyr Glu Leu Thr Lys
       130                 135                 140
   Met Cys Thr Ile Arg Met Ser Phe Val Lys Gly Trp Gly Ala Glu Tyr
   145                 150                 155                 160
   His Arg Gln Asp Val Thr Ser Thr Pro Cys Trp Ile Glu Ile His Leu
                   165                 170                 175
   His Gly Pro
<210> 15
   <211> 178
   <212> PRT
   <213> Drosophila melanogaster
<400> 15
   Tyr His Glu Pro Ala Phe Trp Cys Ser Ile Ser Tyr Tyr Glu Leu Asn
   1               5                   10                  15
   Thr Arg Val Gly Glu Thr Phe His Ala Ser Gln Pro Ser Ile Thr Val
   20 25 30
   Asp Gly Phe Thr Asp Pro Ser Asn Ser Glu Arg Phe Cys Leu Gly Leu
   35 40 45
   Leu Ser Asn Val Asn Arg Asn Glu Val Val Glu Gln Thr Arg Arg His
   50 55 60
   Ile Gly Lys Gly Val Arg Leu Tyr Tyr Ile Gly Gly Glu Val Phe Ala
   65 70 75 80
   Glu Cys Leu Ser Asp Ser Ser Ile Phe Val Gln Ser Pro Asn Cys Asn
   85 90 95
   Gln Arg Tyr Gly Trp His Pro Ala Thr Val Cys Lys Ile Pro Pro Gly
   100 105 110
   Cys Asn Leu Lys Ile Phe Asn Asn Gln Glu Phe Ala Ala Leu Leu Ser
   115 120 125
   Gln Ser Val Ser Gln Gly Phe Glu Ala Val Tyr Gln Leu Thr Arg Met
   130 135 140
   Cys Thr Ile Arg Met Ser Phe Val Lys Gly Trp Gly Ala Glu Tyr Arg
   145 150 155 160
   Arg Gln Thr Val Thr Ser Thr Pro Cys Trp Ile Glu Leu His Leu Asn
   165 170 175
   Gly Pro
<210> 16
   <211> 179
   <212> PRT
   <213> Homo sapiens
<400> 16
   Tyr Glu Glu Pro Lys His Trp Cys Ser Ile Val Tyr Tyr Glu Leu Asn
   1 5 10 15
   Asn Arg Val Gly Glu Ala Phe His Ala Ser Ser Thr Ser Val Leu Val
   20 25 30
   Asp Gly Phe Thr Asp Pro Ser Asn Asn Lys Asn Arg Phe Cys Leu Gly
   35 40 45
   Leu Leu Ser Asn Val Asn Arg Asn Ser Thr Ile Glu Asn Thr Arg Arg
   50 55 60
   His Ile Gly Lys Gly Val His Leu Tyr Tyr Val Gly Gly Glu Val Tyr
   65 70 75 80
   Ala Glu Cys Leu Ser Asp Ser Ser Ile Phe Val Gln Ser Arg Asn Cys
   85 90 95
   Asn Tyr His His Gly Phe His Pro Thr Thr Val Cys Lys Ile Pro Ser
   100 105 110
   Gly Cys Ser Leu Lys Ile Phe Asn Asn Gln Glu Phe Ala Gln Leu Leu
   115 120 125
   Ala Gln Ser Val Asn His Gly Phe Glu Thr Val Tyr Glu Leu Thr Lys
   130 135 140
   Met Cys Thr Ile Arg Met Ser Phe Val Lys Gly Trp Gly Ala Glu Tyr
   145 150 155 160
   His Arg Gln Asp Val Thr Ser Thr Pro Cys Trp Ile Glu Ile His Leu
   165 170 175
   His Gly Pro
<210> 17
   <211> 173
   <212> PRT
   <213> Homo sapiens
<400> 17
   Lys His Trp Cys Ser Ile Val Tyr Tyr Glu Leu Asn Asn Arg Val Gly
   1 5 10 15
   Glu Ala Phe His Ala Ser Ser Thr Ser Val Leu Val Asp Gly Phe Thr
   20 25 30
   Asp Pro Ser Asn Asn Lys Ser Arg Phe Cys Leu Gly Leu Leu Ser Asn
   35 40 45
   Val Asn Arg Asn Ser Thr Ile Glu Asn Thr Arg Arg His Ile Gly Lys
   50 55 60
   Gly Val His Leu Tyr Tyr Val Gly Gly Glu Val Tyr Ala Glu Cys Leu
   65 70 75 80
   Ser Asp Ser Ser Ile Phe Val Gln Ser Arg Asn Cys Asn Phe His His
   85 90 95
   Gly Phe His Pro Thr Thr Val Cys Lys Ile Pro Ser Ser Cys Ser Leu
   100 105 110
   Lys Ile Phe Asn Asn Gln Glu Phe Ala Gln Leu Leu Ala Gln Ser Val
   115 120 125
   Asn His Gly Phe Glu Ala Val Tyr Glu Leu Thr Lys Met Cys Thr Ile
   130 135 140
   Arg Met Ser Phe Val Lys Gly Trp Gly Ala Glu Tyr His Arg Gln Asp
   145 150 155 160
   Val Thr Ser Thr Pro Cys Trp Ile Glu Ile His Leu His
   165 170
<210> 18
   <211> 178
   <212> PRT
   <213> Homo sapiens
<400> 18
   Tyr Ser Glu Pro Ala Phe Trp Cys Ser Ile Ala Tyr Tyr Glu Leu Asn
   1               5                   10                  15
   Gln Arg Val Gly Glu Thr Phe His Ala Ser Gln Pro Ser Leu Thr Val
               20                  25                  30
   Asp Gly Phe Thr Asp Pro Ser Asn Ser Glu Arg Phe Cys Leu Gly Leu
           35                  40                  45
   Leu Ser Asn Val Asn Arg Asn Ala Thr Val Glu Met Thr Arg Arg His
       50                  55                  60
   Ile Gly Arg Gly Val Arg Leu Tyr Tyr Ile Gly Gly Glu Val Phe Ala
   65                  70                  75                  80
   Glu Cys Leu Ser Asp Ser Ala Ile Phe Val Gln Ser Pro Asn Cys Asn
                   85                  90                  95
   Gln Arg Tyr Gly Trp His Pro Ala Thr Val Cys Lys Ile Pro Pro Gly
               100                 105                 110
   Cys Asn Leu Lys Ile Phe Asn Asn Gln Glu Phe Ala Ala Leu Leu Ala
           115                 120                 125
   Gln Ser Val Asn Gln Gly Phe Glu Ala Val Tyr Gln Leu Thr Arg Met
       130                 135                 140
   Cys Thr Ile Arg Met Ser Phe Val Lys Gly Trp Gly Ala Glu Tyr Arg
   145                 150                 155                 160
   Arg Gln Thr Val Thr Ser Thr Pro Cys Trp Ile Glu Leu His Leu Asn
                   165                 170                 175
   Gly Pro
<210> 19
   <211> 178
   <212> PRT
   <213> Homo sapiens
<400> 19
   Tyr Cys Glu Pro Ala Phe Trp Cys Ser Ile Ser Tyr Tyr Glu Leu Asn
   1 5 10 15
   Gln Arg Val Gly Glu Thr Phe His Ala Ser Gln Pro Ser Met Thr Val
   20 25 30
   Asp Gly Phe Thr Asp Pro Ser Asn Ser Glu Arg Phe Cys Leu Gly Leu
   35 40 45
   Leu Ser Asn Val Asn Arg Asn Ala Ala Val Glu Leu Thr Arg Arg His
   50 55 60
   Ile Gly Arg Gly Val Arg Leu Tyr Tyr Ile Gly Gly Glu Val Phe Ala
   65 70 75 80
   Glu Cys Leu Ser Asp Ser Ala Ile Phe Val Gln Ser Pro Asn Cys Asn
   85 90 95
   Gln Arg Tyr Gly Trp His Pro Ala Thr Val Cys Lys Ile Pro Pro Gly
   100 105 110
   Cys Asn Leu Lys Ile Phe Asn Asn Gln Glu Phe Ala Ala Leu Leu Ala
   115 120 125
   Gln Ser Val Asn Gln Gly Phe Glu Ala Val Tyr Gln Leu Thr Arg Met
   130 135 140
   Cys Thr Ile Arg Met Ser Phe Val Lys Gly Trp Gly Ala Glu Tyr Arg
   145 150 155 160
   Arg Gln Thr Val Thr Ser Thr Pro Cys Trp Ile Glu Leu His Leu Asn
   165 170 175
   Gly Pro
<210> 20
   <211> 870
   <212> PRT
   <213> Artificial Sequence
<220>
   <223> TIPF reporter
<400> 20
   Met Ala Pro Lys Ser Arg Lys Lys Lys Ala His Ala Arg Ser Leu Thr
   1 5 10 15
   Cys Tyr Cys Asp Gly Ser Cys Pro Asp Asn Val Ser Asn Gly Thr Cys
   20 25 30
   Glu Thr Arg Pro Gly Gly Ser Cys Phe Ser Ala Val Gln Gln Leu Tyr
   35 40 45
   Asp Glu Thr Thr Gly Met Tyr Glu Glu Glu Arg Thr Tyr Gly Cys Met
   50 55 60
   Pro Pro Glu Asp Asn Gly Gly Phe Leu Met Cys Lys Val Ala Ala Val
   65 70 75 80
   Pro His Leu His Gly Lys Asn Ile Val Cys Cys Asp Lys Glu Asp Phe
   85 90 95
   Cys Asn Arg Asp Leu Tyr Pro Thr Tyr Thr Pro Lys Leu Thr Thr Pro
   100 105 110
   Ala Pro Asp Leu Pro Val Ser Ser Glu Ser Leu His Thr Leu Ala Val
   115 120 125
   Phe Gly Ser Ile Ile Ile Ser Leu Ser Val Phe Met Leu Ile Val Ala
   130 135 140
   Ser Leu Cys Phe Thr Tyr Lys Arg Arg Glu Lys Leu Arg Lys Gln Pro
   145 150 155 160
   Arg Leu Ile Asn Ser Met Cys Asn Ser Gln Leu Ser Pro Leu Ser Gln
   165 170 175
   Leu Val Glu Gln Ser Ser Gly Ser Gly Ser Gly Leu Pro Leu Leu Val
   180 185 190
   Gln Arg Thr Ile Ala Lys Gln Ile Gln Met Val Arg Leu Val Gly Lys
   195 200 205
   Gly Arg Tyr Gly Glu Val Trp Leu Ala Lys Trp Arg Asp Glu Arg Val
   210 215 220
   Ala Val Lys Thr Phe Phe Thr Thr Glu Glu Ala Ser Trp Phe Arg Glu
   225 230 235 240
   Thr Glu Ile Tyr Gln Thr Val Leu Met Arg His Asp Asn Ile Leu Gly
   245 250 255
   Phe Ile Ala Ala Asp Ile Lys Gly Asn Gly Ser Trp Thr Gln Met Leu
   260 265 270
   Leu Ile Thr Asp Tyr His Glu Met Gly Ser Leu His Asp Tyr Leu Ser
   275 280 285
   Met Ser Val Ile Asn Pro Gln Lys Leu Gln Leu Leu Ala Phe Ser Leu
   290 295 300
   Ala Ser Gly Leu Ala His Leu His Asp Glu Ile Phe Gly Thr Pro Gly
   305                 310                 315                 320
   Lys Pro Ala Ile Ala His Arg Asp Ile Lys Ser Lys Asn Ile Leu Val
                   325                 330                 335
   Lys Arg Asn Gly Gln Cys Ala Ile Ala Asp Phe Gly Leu Ala Val Lys
               340                 345                 350
   Tyr Asn Ser Glu Leu Asp Val Ile His Ile Ala Gln Asn Pro Arg Val
           355                 360                 365
   Gly Thr Arg Arg Tyr Met Ala Pro Glu Val Leu Ser Gln Gln Leu Asp
       370                 375                 380
   Pro Lys Gln Phe Glu Glu Phe Lys Arg Ala Asp Met Tyr Ser Val Gly
   385                 390                 395                 400
   Leu Val Leu Trp Glu Met Thr Arg Arg Cys Tyr Thr Pro Val Ser Gly
                   405                 410                 415
   Thr Lys Thr Thr Thr Cys Glu Asp Tyr Ala Leu Pro Tyr His Asp Val
               420                 425                 430
   Val Pro Ser Asp Pro Thr Phe Glu Asp Met His Ala Val Val Cys Val
           435                 440                 445
   Lys Gly Phe Arg Pro Pro Ile Pro Ser Arg Trp Gln Glu Asp Asp Val
       450                 455                 460
   Leu Ala Thr Val Ser Lys Ile Met Gln Glu Cys Trp His Pro Asn Pro
   465                 470                 475                 480
   Thr Val Arg Leu Thr Ala Leu Arg Val Lys Lys Thr Leu Gly Arg Leu
                   485                 490                 495
   Glu Thr Asp Cys Leu Ile Asp Val Pro Ile Lys Ile Val Ser Ala Gly
               500                 505                 510
   Tyr Asn Ser Thr Asn Val Tyr Ile Met Ala Asp Lys Gln Lys Asn Gly
           515                 520                 525
   Ile Lys Ala Asn Phe Lys Ile Arg His Asn Ile Glu Asp Gly Gly Val
       530                 535                 540
   Gln Leu Ala Asp His Tyr Gln Gln Asn Thr Pro Ile Gly Asp Gly Pro
   545                 550                 555                 560
   Val Leu Leu Pro Asp Asn His Tyr Leu Ser Phe Gln Ser Ala Leu Ser
                   565                 570                 575
   Lys Asp Pro Asn Glu Lys Arg Asp His Met Val Leu Leu Glu Phe Val
   580 585 590
   Thr Ala Ala Gly Ile Thr Leu Gly Met Asp Glu Leu Tyr Lys Val Asp
   595 600 605
   Gly Gly Ser Gly Gly Thr Gly Val Ser Lys Gly Glu Glu Leu Phe Thr
   610 615 620
   Gly Val Val Pro Ile Leu Val Glu Leu Asp Gly Asp Val Asn Gly His
   625 630 635 640
   Lys Phe Ser Val Ser Gly Glu Gly Glu Gly Asp Ala Thr Tyr Gly Lys
   645 650 655
   Leu Thr Leu Lys Leu Ile Cys Thr Thr Gly Lys Leu Pro Val Pro Trp
   660 665 670
   Pro Thr Leu Val Thr Thr Phe Gly Tyr Gly Leu Lys Cys Phe Ala Arg
   675 680 685
   Tyr Pro Asp His Met Lys Gln His Asp Phe Phe Lys Ser Ala Met Pro
   690 695 700
   Glu Gly Tyr Val Gln Glu Arg Thr Ile Phe Phe Lys Asp Asp Gly Asn
   705 710 715 720
   Tyr Lys Thr Arg Ala Glu Val Lys Phe Glu Gly Asp Thr Leu Val Asn
   725 730 735
   Arg Ile Glu Leu Lys Gly Ile Asp Phe Lys Glu Asp Gly Asn Ile Leu
   740 745 750
   Gly His Lys Leu Glu Tyr Asn Gly Thr Gly Met Gly Val Gln Val Val
   755 760 765
   Pro Ile Ala Pro Gly Asp Gly Ser Thr Tyr Pro Lys Asn Gly Gln Lys
   770 775 780
   Val Thr Val His Tyr Thr Gly Thr Leu Asp Asp Gly Thr Lys Phe Asp
   785 790 795 800
   Ser Ser Arg Asp Arg Asn Lys Pro Phe Lys Phe Thr Ile Gly Lys Gly
   805 810 815
   Glu Val Ile Arg Gly Trp Asp Glu Gly Val Ala Gln Leu Ser Val Gly
   820 825 830
   Gln Arg Ala Lys Leu Ile Cys Ser Pro Asp Tyr Ala Tyr Gly Ser Arg
   835 840 845
   Gly His Pro Gly Val Ile Pro Pro Asn Ser Thr Leu Thr Phe Asp Val
   850 855 860
   Glu Leu Leu Lys Val Glu
   865 870
<210> 21
   <211> 888
   <212> PRT
   <213> Artificial Sequence
<220>
   <223> Zebrafish ALK3 reporter
<400> 21
   Met Arg Gln Leu Leu Phe Ile Thr Val Val Leu Thr Gly Val Cys Leu
   1               5                   10                  15
   Leu Leu Thr Leu Cys Ser Gly Ala Gly Gln Asn Pro Asp His Val Leu
               20                  25                  30
   Gln Gly Thr Gly Val Lys Leu Asp Ser Arg Arg Pro Gly Asp Asp Ser
           35                  40                  45
   Thr Ile Ala Pro Glu Asp Ala Ala Arg Phe Leu Ser Cys His Cys Ser
       50                  55                  60
   Gly His Cys Pro Asp Asp Ala Lys Asn Asn Thr Cys Glu Thr Asn Gly
   65                  70                  75                  80
   Gln Cys Phe Ala Ile Asn Glu Glu Asp Glu Asn Gly Asp Val Ile Leu
                   85                  90                  95
   Ser Ser Gly Cys Met Lys Tyr Glu Gly Ser His Phe Gln Cys Lys Asp
               100                 105                 110
   Ser Gln Phe Ala Gln Thr Arg Arg Thr Ile Glu Cys Cys Gln Phe Asp
           115                 120                 125
   Phe Cys Asn Gln Asp Leu Lys Pro Glu Leu Pro Pro Arg Asp Ser Glu
       130                 135                 140
   Pro Pro Asp Pro His Trp Leu Ala Phe Leu Ile Ser Val Thr Val Cys
   145                 150                 155                 160
   Phe Cys Ala Leu Ile Cys Val Thr Val Ile Cys Tyr Tyr Arg Tyr Lys
                   165                 170                 175
   Trp Gln Thr Glu Arg Gln Arg Tyr His Arg Asp Leu Glu Gln Asp Glu
               180                 185                 190
   Ala Phe Ile Pro Ala Gly Glu Ser Leu Lys Asp Leu Ile Asn Gln Ser
           195                 200                 205
   Gln Thr Ser Gly Ser Gly Ser Gly Leu Pro Leu Leu Val Gln Arg Thr
   210 215 220
   Ile Arg Lys Gln Ile Gln Thr Val Arg Met Ile Gly Lys Gly Arg Tyr
   225 230 235 240
   Gly Glu Val Trp Leu Gly Arg Trp Arg Gly Glu Lys Val Ala Val Lys
   245 250 255
   Val Phe Phe Thr Arg Glu Glu Ala Ser Trp Phe Arg Glu Thr Glu Ile
   260 265 270
   Tyr Gln Thr Val Leu Met Arg His Glu Asn Ile Leu Gly Phe Ile Ala
   275 280 285
   Ala Asp Ile Asn Gly Thr Gly Ala Ser Thr Gln Leu Tyr Leu Ile Thr
   290 295 300
   Asp Tyr His Glu Asn Gly Ser Leu Tyr Asp Tyr Leu Lys Phe Thr Thr
   305 310 315 320
   Leu Asp Thr Gln Ala Leu Leu Arg Leu Ala Phe Ser Ala Ala Cys Gly
   325 330 335
   Leu Cys His Leu His Thr Glu Ile Tyr Gly Thr Gln Gly Lys Pro Ala
   340 345 350
   Ile Ala His Arg Asp Leu Lys Ser Lys Asn Ile Leu Ile Lys Lys Asn
   355 360 365
   Gly Thr Cys Cys Ile Ala Asp Leu Gly Leu Ala Val Lys Phe Asn Ser
   370 375 380
   Asp Thr Asn Glu Val Asp Leu Pro Leu Ser Thr Arg Met Gly Thr Arg
   385 390 395 400
   Arg Tyr Met Ala Pro Glu Val Leu Asp Glu Thr Leu Asn Lys Asn His
   405 410 415
   Phe Gln Ala Tyr Ile Met Ala Asp Ile Tyr Ser Tyr Gly Leu Val Ile
   420 425 430
   Trp Glu Met Ala Arg Arg Cys Val Thr Gly Gly Ile Val Glu Glu Tyr
   435 440 445
   His Val Pro Tyr Tyr Glu Met Val Pro Ser Asp Pro Ser Tyr Glu Asp
   450 455 460
   Met Leu Glu Val Val Cys Val Lys Gly Leu Arg Pro Thr Val Ser Asn
   465 470 475 480
   Arg Trp Asn Ser Asp Glu Cys Leu Arg Ala Met Leu Lys Leu Met Ser
   485 490 495
   Glu Cys Trp Ala His Asn Pro Ala Ser Arg Leu Thr Ile Leu Arg Val
   500 505 510
   Lys Lys Thr Leu Ala Lys Met Val Glu Ser Gln Asp Ile Lys Ile Tyr
   515 520 525
   Ala Gly Tyr Asn Ser Thr Asn Val Tyr Ile Met Ala Asp Lys Gln Lys
   530 535 540
   Asn Gly Ile Lys Ala Asn Phe Lys Ile Arg His Asn Ile Glu Asp Gly
   545 550 555 560
   Gly Val Gln Leu Ala Asp His Tyr Gln Gln Asn Thr Pro Ile Gly Asp
   565 570 575
   Gly Pro Val Leu Leu Pro Asp Asn His Tyr Leu Ser Phe Gln Ser Ala
   580 585 590
   Leu Ser Lys Asp Pro Asn Glu Lys Arg Asp His Met Val Leu Leu Glu
   595 600 605
   Phe Val Thr Ala Ala Gly Ile Thr Leu Gly Met Asp Glu Leu Tyr Lys
   610 615 620
   Val Asp Gly Gly Ser Gly Gly Thr Gly Val Ser Lys Gly Glu Glu Leu
   625 630 635 640
   Phe Thr Gly Val Val Pro Ile Leu Val Glu Leu Asp Gly Asp Val Asn
   645 650 655
   Gly His Lys Phe Ser Val Ser Gly Glu Gly Glu Gly Asp Ala Thr Tyr
   660 665 670
   Gly Lys Leu Thr Leu Lys Leu Ile Cys Thr Thr Gly Lys Leu Pro Val
   675 680 685
   Pro Trp Pro Thr Leu Val Thr Thr Phe Gly Tyr Gly Leu Lys Cys Phe
   690 695 700
   Ala Arg Tyr Pro Asp His Met Lys Gln His Asp Phe Phe Lys Ser Ala
   705 710 715 720
   Met Pro Glu Gly Tyr Val Gln Glu Arg Thr Ile Phe Phe Lys Asp Asp
   725 730 735
   Gly Asn Tyr Lys Thr Arg Ala Glu Val Lys Phe Glu Gly Asp Thr Leu
   740 745 750
   Val Asn Arg Ile Glu Leu Lys Gly Ile Asp Phe Lys Glu Asp Gly Asn
   755 760 765
   Ile Leu Gly His Lys Leu Glu Tyr Asn Gly Thr Gly Met Gly Val Gln
       770                 775                 780
   Val Val Pro Ile Ala Pro Gly Asp Gly Ser Thr Tyr Pro Lys Asn Gly
   785                 790                 795                 800
   Gln Lys Val Thr Val His Tyr Thr Gly Thr Leu Asp Asp Gly Thr Lys
                   805                 810                 815
   Phe Asp Ser Ser Arg Asp Arg Asn Lys Pro Phe Lys Phe Thr Ile Gly
               820                 825                 830
   Lys Gly Glu Val Ile Arg Gly Trp Asp Glu Gly Val Ala Gln Leu Ser
           835                 840                 845
   Val Gly Gln Arg Ala Lys Leu Ile Cys Ser Pro Asp Tyr Ala Tyr Gly
       850                 855                 860
   Ser Arg Gly His Pro Gly Val Ile Pro Pro Asn Ser Thr Leu Thr Phe
   865                 870                 875                 880
   Asp Val Glu Leu Leu Lys Val Glu
                   885
<210> 22
   <211> 5890
   <212> DNA
   <213> Artificial Sequence
<220>
   <223> Cloning vector
<400> 22
   cgccattctg cctggggacg tcggagcaag cttgatttag gtgacactat agaatacaag     60
   ctacttgttc tttttgcagg atcccatcga ttcgaattcc tgcaggctac aacagcacca    120
   acgtctatat catggccgac aagcagaaga acggcatcaa ggccaacttc aagatccgcc    180
   acaacatcga ggacggcggc gtgcagctcg ccgaccacta ccagcagaac acccccatcg    240
   gcgacggccc cgtgctgctg cccgacaacc actacctgag cttccagtcc gccctgagca    300
   aagaccccaa cgagaagcgc gatcacatgg tcctgctgga gttcgtgacc gccgccggga    360
   tcactctcgg catggacgag ctgtacaagg tcgacggtgg cagcggtggc accggtgtga    420
   gcaagggcga ggagctgttc accggggtgg tgcccatcct ggtcgagctg gacggcgacg    480
   taaacggcca caagttcagc gtgtccggcg agggcgaggg cgatgccacc tacggcaagc    540
   tgaccctgaa gctcatctgc accaccggca agctgcccgt gccctggccc accctcgtga    600
   ccaccttcgg ctacggcctg aagtgcttcg cccgctaccc cgaccacatg aagcagcacg    660
   acttcttcaa gtccgccatg cccgaaggct acgtccagga gcgcaccatc ttcttcaagg    720
   acgacggcaa ctacaagacc cgcgccgagg tgaagttcga gggcgacacc ctggtgaacc    780
   gcatcgagct gaagggcatc gacttcaagg aggacggcaa catcctgggg cacaagctgg    840
   agtacaacgg taccgggatg ggcgtacaag tagttccaat tgctcctggt gatggcagca 900
   cctatcccaa gaatggccaa aaggtcacgg tccactacac cggcaccctg gacgatggca 960
   ccaagttcga ttcgtcgcgc gaccgcaaca agccattcaa gttcaccatc ggcaagggcg 1020
   aggtcatccg tggctgggat gagggagttg cccagttgag cgtcggccag cgcgccaagc 1080
   tgatttgctc gccggactat gcctacggta gccgtggcca ccccggcgtc attccgccca 1140
   actccaccct caccttcgac gtcgagctgc tcaaggtcga ataggcagct gtttgtagtc 1200
   tcgttttagg tttaacctaa gttgcattta gttttctgtt tttgttcaac ttctgttgaa 1260
   tacgcagttc cgaccttttg tccggaaatt catagttagg ttctaatcgt aagttgcttg 1320
   tacgtgagcg gagtaaagca gaggatcctt cagaggtgcc tatactacgg ttacttaaac 1380
   cgtcgcactg attgtaagct agagtttaga tataagttgg caggagctag ttactaaggc 1440
   cccttgcaaa atctttggtt agaaaaatgc tttaactatt tgagaaatat agggagtatt 1500
   ttggatgaca cctattcatg tcgatgtaca taaatatgag atcaaaacaa aaaatcaatc 1560
   gaaagaatcg aaatttatat gtaacataca ttaatttttg tgccagcttt tgtacgcttt 1620
   aactaaacga gttaagtcgc ccattctttg acccccgagg ctttagcctc aacatagaac 1680
   atgtccttat atgttttgtt tgtatctagt gtttaagtgt aaactttgca gactttgtgc 1740
   aagtaaatgc tgccttttgt aaaatccacg gaattagttg ccttaagaca aagtgtaaag 1800
   acaggagcca acagacagga gcccgactcc cttcctcccc ttttagtagc acatattttt 1860
   gtatttataa ccagagatat atagaactgg cagttgcgta tctcgagcct ctagaactat 1920
   agtgagtcgt attacgtaga tccagacatg ataagataca ttgatgagtt tggacaaacc 1980
   acaactagaa tgcagtgaaa aaaatgcttt atttgtgaaa tttgtgatgc tattgcttta 2040
   tttgtaacca ttataagctg caataaacaa gttaacaaca acaattgcat tcattttatg 2100
   tttcaggttc agggggaggt gtgggaggtt ttttaattcg cggccgcggc gccaatgcat 2160
   tgggcccggt acccagcttt tgttcccttt agtgagggtt aattgcgcgc ttggcgtaat 2220
   catggtcata gctgtttcct gtgtgaaatt gttatccgct cacaattcca cacaacatac 2280
   gagccggaag cataaagtgt aaagcctggg gtgcctaatg agtgagctaa ctcacattaa 2340
   ttgcgttgcg ctcactgccc gctttccagt cgggaaacct gtcgtgccag ctgcattaat 2400
   gaatcggcca acgcgcgggg agaggcggtt tgcgtattgg gcgctcttcc gcttcctcgc 2460
   tcactgactc gctgcgctcg gtcgttcggc tgcggcgagc ggtatcagct cactcaaagg 2520
   cggtaatacg gttatccaca gaatcagggg ataacgcagg aaagaacatg tgagcaaaag 2580
   gccagcaaaa ggccaggaac cgtaaaaagg ccgcgttgct ggcgtttttc cataggctcc 2640
   gcccccctga cgagcatcac aaaaatcgac gctcaagtca gaggtggcga aacccgacag 2700
   gactataaag ataccaggcg tttccccctg gaagctccct cgtgcgctct cctgttccga 2760
   ccctgccgct taccggatac ctgtccgcct ttctcccttc gggaagcgtg gcgctttctc 2820
   atagctcacg ctgtaggtat ctcagttcgg tgtaggtcgt tcgctccaag ctgggctgtg 2880
   tgcacgaacc ccccgttcag cccgaccgct gcgccttatc cggtaactat cgtcttgagt 2940
   ccaacccggt aagacacgac ttatcgccac tggcagcagc cactggtaac aggattagca 3000
   gagcgaggta tgtaggcggt gctacagagt tcttgaagtg gtggcctaac tacggctaca 3060
   ctagaaggac agtatttggt atctgcgctc tgctgaagcc agttaccttc ggaaaaagag 3120
   ttggtagctc ttgatccggc aaacaaacca ccgctggtag cggtggtttt tttgtttgca 3180
   agcagcagat tacgcgcaga aaaaaaggat ctcaagaaga tcctttgatc ttttctacgg 3240
   ggtctgacgc tcagtggaac gaaaactcac gttaagggat tttggtcatg agattatcaa 3300
   aaaggatctt cacctagatc cttttaaatt aaaaatgaag ttttaaatca atctaaagta 3360
   tatatgagta aacttggtct gacagttacc aatgcttaat cagtgaggca cctatctcag 3420
   cgatctgtct atttcgttca tccatagttg cctgactccc cgtcgtgtag ataactacga 3480
   tacgggaggg cttaccatct ggccccagtg ctgcaatgat accgcgagac ccacgctcac 3540
   cggctccaga tttatcagca ataaaccagc cagccggaag ggccgagcgc agaagtggtc 3600
   ctgcaacttt atccgcctcc atccagtcta ttaattgttg ccgggaagct agagtaagta 3660
   gttcgccagt taatagtttg cgcaacgttg ttgccattgc tacaggcatc gtggtgtcac 3720
   gctcgtcgtt tggtatggct tcattcagct ccggttccca acgatcaagg cgagttacat 3780
   gatcccccat gttgtgcaaa aaagcggtta gctccttcgg tcctccgatc gttgtcagaa 3840
   gtaagttggc cgcagtgtta tcactcatgg ttatggcagc actgcataat tctcttactg 3900
   tcatgccatc cgtaagatgc ttttctgtga ctggtgagta ctcaaccaag tcattctgag 3960
   aatagtgtat gcggcgaccg agttgctctt gcccggcgtc aatacgggat aataccgcgc 4020
   cacatagcag aactttaaaa gtgctcatca ttggaaaacg ttcttcgggg cgaaaactct 4080
   caaggatctt accgctgttg agatccagtt cgatgtaacc cactcgtgca cccaactgat 4140
   cttcagcatc ttttactttc accagcgttt ctgggtgagc aaaaacagga aggcaaaatg 4200
   ccgcaaaaaa gggaataagg gcgacacgga aatgttgaat actcatactc ttcctttttc 4260
   aatattattg aagcatttat cagggttatt gtctcatgag cggatacata tttgaatgta 4320
   tttagaaaaa taaacaaata ggggttccgc gcacatttcc ccgaaaagtg ccacctaaat 4380
   tgtaagcgtt aatattttgt taaaattcgc gttaaatttt tgttaaatca gctcattttt 4440
   taaccaatag gccgaaatcg gcaaaatccc ttataaatca aaagaataga ccgagatagg 4500
   gttgagtgtt gttccagttt ggaacaagag tccactatta aagaacgtgg actccaacgt 4560
   caaagggcga aaaaccgtct atcagggcga tggcccacta cgtgaaccat caccctaatc 4620
   aagttttttg gggtcgaggt gccgtaaagc actaaatcgg aaccctaaag ggagcccccg 4680
   atttagagct tgacggggaa agccggcgaa cgtggcgaga aaggaaggga agaaagcgaa 4740
   aggagcgggc gctagggcgc tggcaagtgt agcggtcacg ctgcgcgtaa ccaccacacc 4800
   cgccgcgctt aatgcgccgc tacagggcgc gtcccattcg ccattcaggc tgcgcaactg 4860
   ttgggaaggg cgatcggtgc gggcctcttc gctattacgc cagtcgacca tagccaattc 4920
   aatatggcgt atatggactc atgccaattc aatatggtgg atctggacct gtgccaattc 4980
   aatatggcgt atatggactc gtgccaattc aatatggtgg atctggaccc cagccaattc 5040
   aatatggcgg acttggcacc atgccaattc aatatggcgg acttggcact gtgccaactg 5100
   gggaggggtc tacttggcac ggtgccaagt ttgaggaggg gtcttggccc tgtgccaagt 5160
   ccgccatatt gaattggcat ggtgccaata atggcggcca tattggctat atgccaggat 5220
   caatatatag gcaatatcca atatggccct atgccaatat ggctattggc caggttcaat 5280
   actatgtatt ggccctatgc catatagtat tccatatatg ggttttccta ttgacgtaga 5340
   tagcccctcc caatgggcgg tcccatatac catatatggg gcttcctaat accgcccata 5400
   gccactcccc cattgacgtc aatggtctct atatatggtc tttcctattg acgtcatatg 5460
   ggcggtccta ttgacgtata tggcgcctcc cccattgacg tcaattacgg taaatggccc 5520
   gcctggctca atgcccattg acgtcaatag gaccacccac cattgacgtc aatgggatgg 5580
   ctcattgccc attcatatcc gttctcacgc cccctattga cgtcaatgac ggtaaatggc 5640
   ccacttggca gtacatcaat atctattaat agtaacttgg caagtacatt actattggaa 5700
   ggacgccagg gtacattggc agtactccca ttgacgtcaa tggcggtaaa tggcccgcga 5760
   tggctgccaa gtacatcccc attgacgtca atggggaggg gcaatgacgc aaatgggcgt 5820
   tccattgacg taaatgggcg gtaggcgtgc ctaatgggag gtctatataa gcaatgctcg 5880
   tttagggaac 5890
<210> 23
   <211> 238
   <212> PRT
   <213> Aequorea victoria
<400> 23
   Met Ser Lys Gly Glu Glu Leu Phe Thr Gly Val Val Pro Ile Leu Val
   1               5                   10                  15
   Glu Leu Asp Gly Asp Val Asn Gly Gln Lys Phe Ser Val Ser Gly Glu
               20                  25                  30
   Gly Glu Gly Asp Ala Thr Tyr Gly Lys Leu Thr Leu Lys Phe Ile Cys
           35                  40                  45
   Thr Thr Gly Lys Leu Pro Val Pro Trp Pro Thr Leu Val Thr Thr Phe
       50                  55                  60
   Ser Tyr Gly Val Gln Cys Phe Ser Arg Tyr Pro Asp His Met Lys Gln
   65                  70                  75                  80
   His Asp Phe Phe Lys Ser Ala Met Pro Glu Gly Tyr Val Gln Glu Arg
                   85                  90                  95
   Thr Ile Phe Tyr Lys Asp Asp Gly Asn Tyr Lys Thr Arg Ala Glu Val
               100                 105                 110
   Lys Phe Glu Gly Asp Thr Leu Val Asn Arg Ile Glu Leu Lys Gly Ile
           115                 120                 125
   Asp Phe Lys Glu Asp Gly Asn Ile Leu Gly His Lys Met Glu Tyr Asn
       130                 135                 140
   Tyr Asn Ser His Asn Val Tyr Ile Met Ala Asp Lys Pro Lys Asn Gly
   145                 150                 155                 160
   Ile Lys Val Asn Phe Lys Ile Arg His Asn Ile Lys Asp Gly Ser Val
                   165                 170                 175
   Gln Leu Ala Asp His Tyr Gln Gln Asn Thr Pro Ile Gly Asp Gly Pro
               180                 185                 190
   Val Leu Leu Pro Asp Asn His Tyr Leu Ser Thr Gln Ser Ala Leu Ser
           195                 200                 205
   Lys Asp Pro Asn Glu Lys Arg Asp His Met Ile Leu Leu Glu Phe Val
       210                 215                 220
   Thr Ala Ala Gly Ile Thr His Gly Met Asp Glu Leu Tyr Lys
   225                 230                 235
<210> 24
   <211> 869
   <212> PRT
   <213> Artificial Sequence
<220>
   <223> Human ALK2 reporter
<400> 24
   Met Val Asp Gly Val Met Ile Leu Pro Val Leu Ile Met Ile Ala Leu
   1               5                   10                  15
   Pro Ser Pro Ser Met Glu Asp Glu Lys Pro Lys Val Asn Pro Lys Leu
               20                  25                  30
   Tyr Met Cys Val Cys Glu Gly Leu Ser Cys Gly Asn Glu Asp His Cys
           35                  40                  45
   Glu Gly Gln Gln Cys Phe Ser Ser Leu Ser Ile Asn Asp Gly Phe His
       50                  55                  60
   Val Tyr Gln Lys Gly Cys Phe Gln Val Tyr Glu Gln Gly Lys Met Thr
   65                  70                  75                  80
   Cys Lys Thr Pro Pro Ser Pro Gly Gln Ala Val Glu Cys Cys Gln Gly
                   85                  90                  95
   Asp Trp Cys Asn Arg Asn Ile Thr Ala Gln Leu Pro Thr Lys Gly Lys
               100                 105                 110
   Ser Phe Pro Gly Thr Gln Asn Phe His Leu Glu Val Gly Leu Ile Ile
           115                 120                 125
   Leu Ser Val Val Phe Ala Val Cys Leu Leu Ala Cys Leu Leu Gly Val
       130                 135                 140
   Ala Leu Arg Lys Phe Lys Arg Arg Asn Gln Glu Arg Leu Asn Pro Arg
   145                 150                 155                 160
   Asp Val Glu Tyr Gly Thr Ile Glu Gly Leu Ile Thr Thr Asn Val Gly
                   165                 170                 175
   Asp Ser Thr Leu Ala Asp Leu Leu Asp His Ser Cys Thr Ser Gly Ser
               180                 185                 190
   Gly Ser Gly Leu Pro Phe Leu Val Gln Arg Thr Val Ala Arg Gln Ile
           195                 200                 205
   Thr Leu Leu Glu Cys Val Gly Lys Gly Arg Tyr Gly Glu Val Trp Arg
       210                 215                 220
   Gly Ser Trp Gln Gly Glu Asn Val Ala Val Lys Ile Phe Ser Ser Arg
   225                 230                 235                 240
   Asp Glu Lys Ser Trp Phe Arg Glu Thr Glu Leu Tyr Asn Thr Val Met
                   245                 250                 255
   Leu Arg His Glu Asn Ile Leu Gly Phe Ile Ala Ser Asp Met Thr Ser
               260                 265                 270
   Arg His Ser Ser Thr Gln Leu Trp Leu Ile Thr His Tyr His Glu Met
           275                 280                 285
   Gly Ser Leu Tyr Asp Tyr Leu Gln Leu Thr Thr Leu Asp Thr Val Ser
       290                 295                 300
   Cys Leu Arg Ile Val Leu Ser Ile Ala Ser Gly Leu Ala His Leu His
   305                 310                 315                 320
   Ile Glu Ile Phe Gly Thr Gln Gly Lys Pro Ala Ile Ala His Arg Asp
                   325                 330                 335
   Leu Lys Ser Lys Asn Ile Leu Val Lys Lys Asn Gly Gln Cys Cys Ile
               340                 345                 350
   Ala Asp Leu Gly Leu Ala Val Met His Ser Gln Ser Thr Asn Gln Leu
           355                 360                 365
   Asp Val Gly Asn Asn Pro Arg Val Gly Thr Lys Arg Tyr Met Ala Pro
       370                 375                 380
   Glu Val Leu Asp Glu Thr Ile Gln Val Asp Cys Phe Asp Ser Tyr Lys
   385 390 395 400
   Arg Val Asp Ile Trp Ala Phe Gly Leu Val Leu Trp Glu Val Ala Arg
   405 410 415
   Arg Met Val Ser Asn Gly Ile Val Glu Asp Tyr Lys Pro Pro Phe Tyr
   420 425 430
   Asp Val Val Pro Asn Asp Pro Ser Phe Glu Asp Met Arg Lys Val Val
   435 440 445
   Cys Val Asp Gln Gln Arg Pro Asn Ile Pro Asn Arg Trp Phe Ser Asp
   450 455 460
   Pro Thr Leu Thr Ser Leu Ala Lys Leu Met Lys Glu Cys Trp Tyr Gln
   465 470 475 480
   Asn Pro Ser Ala Arg Leu Thr Ala Leu Arg Ile Lys Lys Thr Leu Thr
   485 490 495
   Lys Ile Asp Asn Ser Leu Asp Lys Leu Lys Thr Asp Cys Ala Gly Tyr
   500 505 510
   Asn Ser Thr Asn Val Tyr Ile Met Ala Asp Lys Gln Lys Asn Gly Ile
   515 520 525
   Lys Ala Asn Phe Lys Ile Arg His Asn Ile Glu Asp Gly Gly Val Gln
   530 535 540
   Leu Ala Asp His Tyr Gln Gln Asn Thr Pro Ile Gly Asp Gly Pro Val
   545 550 555 560
   Leu Leu Pro Asp Asn His Tyr Leu Ser Phe Gln Ser Ala Leu Ser Lys
   565 570 575
   Asp Pro Asn Glu Lys Arg Asp His Met Val Leu Leu Glu Phe Val Thr
   580 585 590
   Ala Ala Gly Ile Thr Leu Gly Met Asp Glu Leu Tyr Lys Val Asp Gly
   595 600 605
   Gly Ser Gly Gly Thr Gly Val Ser Lys Gly Glu Glu Leu Phe Thr Gly
   610 615 620
   Val Val Pro Ile Leu Val Glu Leu Asp Gly Asp Val Asn Gly His Lys
   625 630 635 640
   Phe Ser Val Ser Gly Glu Gly Glu Gly Asp Ala Thr Tyr Gly Lys Leu
   645 650 655
   Thr Leu Lys Leu Ile Cys Thr Thr Gly Lys Leu Pro Val Pro Trp Pro
   660 665 670
   Thr Leu Val Thr Thr Phe Gly Tyr Gly Leu Lys Cys Phe Ala Arg Tyr
   675 680 685
   Pro Asp His Met Lys Gln His Asp Phe Phe Lys Ser Ala Met Pro Glu
   690 695 700
   Gly Tyr Val Gln Glu Arg Thr Ile Phe Phe Lys Asp Asp Gly Asn Tyr
   705 710 715 720
   Lys Thr Arg Ala Glu Val Lys Phe Glu Gly Asp Thr Leu Val Asn Arg
   725 730 735
   Ile Glu Leu Lys Gly Ile Asp Phe Lys Glu Asp Gly Asn Ile Leu Gly
   740 745 750
   His Lys Leu Glu Tyr Asn Gly Thr Gly Met Gly Val Gln Val Val Pro
   755 760 765
   Ile Ala Pro Gly Asp Gly Ser Thr Tyr Pro Lys Asn Gly Gln Lys Val
   770 775 780
   Thr Val His Tyr Thr Gly Thr Leu Asp Asp Gly Thr Lys Phe Asp Ser
   785 790 795 800
   Ser Arg Asp Arg Asn Lys Pro Phe Lys Phe Thr Ile Gly Lys Gly Glu
   805 810 815
   Val Ile Arg Gly Trp Asp Glu Gly Val Ala Gln Leu Ser Val Gly Gln
   820 825 830
   Arg Ala Lys Leu Ile Cys Ser Pro Asp Tyr Ala Tyr Gly Ser Arg Gly
   835 840 845
   His Pro Gly Val Ile Pro Pro Asn Ser Thr Leu Thr Phe Asp Val Glu
   850 855 860
   Leu Leu Lys Val Glu
   865
<210> 25
   <211> 892
   <212> PRT
   <213> Artificial Sequence
<220>
   <223> Human ALK3 reporter
<400> 25
   Met Pro Gln Leu Tyr Ile Tyr Ile Arg Leu Leu Gly Ala Tyr Leu Phe
   1 5 10 15
   Ile Ile Ser Arg Val Gln Gly Gln Asn Leu Asp Ser Met Leu His Gly
               20                  25                  30
   Thr Gly Met Lys Ser Asp Ser Asp Gln Lys Lys Ser Glu Asn Gly Val
           35                  40                  45
   Thr Leu Ala Pro Glu Asp Thr Leu Pro Phe Leu Lys Cys Tyr Cys Ser
       50                  55                  60
   Gly His Cys Pro Asp Asp Ala Ile Asn Asn Thr Cys Ile Thr Asn Gly
   65                  70                  75                  80
   His Cys Phe Ala Ile Ile Glu Glu Asp Asp Gln Gly Glu Thr Thr Leu
                   85                  90                  95
   Ala Ser Gly Cys Met Lys Tyr Glu Gly Ser Asp Phe Gln Cys Lys Asp
               100                 105                 110
   Ser Pro Lys Ala Gln Leu Arg Arg Thr Ile Glu Cys Cys Arg Thr Asn
           115                 120                 125
   Leu Cys Asn Gln Tyr Leu Gln Pro Thr Leu Pro Pro Val Val Ile Gly
       130                 135                 140
   Pro Phe Phe Asp Gly Ser Ile Arg Trp Leu Val Leu Leu Ile Ser Met
   145                 150                 155                 160
   Ala Val Cys Ile Ile Ala Met Ile Ile Phe Ser Ser Cys Phe Cys Tyr
                   165                 170                 175
   Lys His Tyr Cys Lys Ser Ile Ser Ser Arg Arg Arg Tyr Asn Arg Asp
               180                 185                 190
   Leu Glu Gln Asp Glu Ala Phe Ile Pro Val Gly Glu Ser Leu Lys Asp
           195                 200                 205
   Leu Ile Asp Gln Ser Gln Ser Ser Gly Ser Gly Ser Gly Leu Pro Leu
       210                 215                 220
   Leu Val Gln Arg Thr Ile Ala Lys Gln Ile Gln Met Val Arg Gln Val
   225                 230                 235                 240
   Gly Lys Gly Arg Tyr Gly Glu Val Trp Met Gly Lys Trp Arg Gly Glu
                   245                 250                 255
   Lys Val Ala Val Lys Val Phe Phe Thr Thr Glu Glu Ala Ser Trp Phe
               260                 265                 270
   Arg Glu Thr Glu Ile Tyr Gln Thr Val Leu Met Arg His Glu Asn Ile
           275                 280                 285
   Leu Gly Phe Ile Ala Ala Asp Ile Lys Gly Thr Gly Ser Trp Thr Gln
   290 295 300
   Leu Tyr Leu Ile Thr Asp Tyr His Glu Asn Gly Ser Leu Tyr Asp Phe
   305 310 315 320
   Leu Lys Cys Ala Thr Leu Asp Thr Arg Ala Leu Leu Lys Leu Ala Tyr
   325 330 335
   Ser Ala Ala Cys Gly Leu Cys His Leu His Thr Glu Ile Tyr Gly Thr
   340 345 350
   Gln Gly Lys Pro Ala Ile Ala His Arg Asp Leu Lys Ser Lys Asn Ile
   355 360 365
   Leu Ile Lys Lys Asn Gly Ser Cys Cys Ile Ala Asp Leu Gly Leu Ala
   370 375 380
   Val Lys Phe Asn Ser Asp Thr Asn Glu Val Asp Val Pro Leu Asn Thr
   385 390 395 400
   Arg Val Gly Thr Lys Arg Tyr Met Ala Pro Glu Val Leu Asp Glu Ser
   405 410 415
   Leu Asn Lys Asn His Phe Gln Pro Tyr Ile Met Ala Asp Ile Tyr Ser
   420 425 430
   Phe Gly Leu Ile Ile Trp Glu Met Ala Arg Arg Cys Ile Thr Gly Gly
   435 440 445
   Ile Val Glu Glu Tyr Gln Leu Pro Tyr Tyr Asn Met Val Pro Ser Asp
   450 455 460
   Pro Ser Tyr Glu Asp Met Arg Glu Val Val Cys Val Lys Arg Leu Arg
   465 470 475 480
   Pro Ile Val Ser Asn Arg Trp Asn Ser Asp Glu Cys Leu Arg Ala Val
   485 490 495
   Leu Lys Leu Met Ser Glu Cys Trp Ala His Asn Pro Ala Ser Arg Leu
   500 505 510
   Thr Ala Leu Arg Ile Lys Lys Thr Leu Ala Lys Met Val Glu Ser Gln
   515 520 525
   Asp Val Lys Ile Ala Gly Tyr Asn Ser Thr Asn Val Tyr Ile Met Ala
   530 535 540
   Asp Lys Gln Lys Asn Gly Ile Lys Ala Asn Phe Lys Ile Arg His Asn
   545 550 555 560
   Ile Glu Asp Gly Gly Val Gln Leu Ala Asp His Tyr Gln Gln Asn Thr
   565 570 575
   Pro Ile Gly Asp Gly Pro Val Leu Leu Pro Asp Asn His Tyr Leu Ser
   580 585 590
   Phe Gln Ser Ala Leu Ser Lys Asp Pro Asn Glu Lys Arg Asp His Met
   595 600 605
   Val Leu Leu Glu Phe Val Thr Ala Ala Gly Ile Thr Leu Gly Met Asp
   610 615 620
   Glu Leu Tyr Lys Val Asp Gly Gly Ser Gly Gly Thr Gly Val Ser Lys
   625 630 635 640
   Gly Glu Glu Leu Phe Thr Gly Val Val Pro Ile Leu Val Glu Leu Asp
   645 650 655
   Gly Asp Val Asn Gly His Lys Phe Ser Val Ser Gly Glu Gly Glu Gly
   660 665 670
   Asp Ala Thr Tyr Gly Lys Leu Thr Leu Lys Leu Ile Cys Thr Thr Gly
   675 680 685
   Lys Leu Pro Val Pro Trp Pro Thr Leu Val Thr Thr Phe Gly Tyr Gly
   690 695 700
   Leu Lys Cys Phe Ala Arg Tyr Pro Asp His Met Lys Gln His Asp Phe
   705 710 715 720
   Phe Lys Ser Ala Met Pro Glu Gly Tyr Val Gln Glu Arg Thr Ile Phe
   725 730 735
   Phe Lys Asp Asp Gly Asn Tyr Lys Thr Arg Ala Glu Val Lys Phe Glu
   740 745 750
   Gly Asp Thr Leu Val Asn Arg Ile Glu Leu Lys Gly Ile Asp Phe Lys
   755 760 765
   Glu Asp Gly Asn Ile Leu Gly His Lys Leu Glu Tyr Asn Gly Thr Gly
   770 775 780
   Met Gly Val Gln Val Val Pro Ile Ala Pro Gly Asp Gly Ser Thr Tyr
   785 790 795 800
   Pro Lys Asn Gly Gln Lys Val Thr Val His Tyr Thr Gly Thr Leu Asp
   805 810 815
   Asp Gly Thr Lys Phe Asp Ser Ser Arg Asp Arg Asn Lys Pro Phe Lys
   820 825 830
   Phe Thr Ile Gly Lys Gly Glu Val Ile Arg Gly Trp Asp Glu Gly Val
   835 840 845
   Ala Gln Leu Ser Val Gly Gln Arg Ala Lys Leu Ile Cys Ser Pro Asp
   850 855 860
   Tyr Ala Tyr Gly Ser Arg Gly His Pro Gly Val Ile Pro Pro Asn Ser
   865 870 875 880
   Thr Leu Thr Phe Asp Val Glu Leu Leu Lys Val Glu
   885 890
<210> 26
   <211> 862
   <212> PRT
   <213> Artificial Sequence
<220>
   <223> Human ALK6 reporter
<400> 26
<210> 27
   <211> 853
   <212> PRT
   <213> Artificial sequence
<220>
   <223> Human ALK7 reporter
<400> 27
<210> 28
   <211> 856
   <212> PRT
   <213> Artificial Sequence
<220>
   <223> Alternative Human ALK6 reporter
<400> 28
   Met Leu Leu Arg Ser Ala Gly Lys Leu Asn Val Gly Thr Lys Lys Glu
   1               5                   10                  15
   Asp Gly Glu Ser Thr Ala Pro Thr Pro Arg Pro Lys Val Leu Arg Cys
               20                  25                  30
   Lys Cys His His His Cys Pro Glu Asp Ser Val Asn Asn Ile Cys Ser
   35 40 45
   Thr Asp Gly Tyr Cys Phe Thr Met Ile Glu Glu Asp Asp Ser Gly Leu
   50 55 60
   Pro Val Val Thr Ser Gly Cys Leu Gly Leu Glu Gly Ser Asp Phe Gln
   65 70 75 80
   Cys Arg Asp Thr Pro Ile Pro His Gln Arg Arg Ser Ile Glu Cys Cys
   85 90 95
   Thr Glu Arg Asn Glu Cys Asn Lys Asp Leu His Pro Thr Leu Pro Pro
   100 105 110
   Leu Lys Asn Arg Asp Phe Val Asp Gly Pro Ile His His Arg Ala Leu
   115 120 125
   Leu Ile Ser Val Thr Val Cys Ser Leu Leu Leu Val Leu Ile Ile Leu
   130 135 140
   Phe Cys Tyr Phe Arg Tyr Lys Arg Gln Glu Thr Arg Pro Arg Tyr Ser
   145 150 155 160
   Ile Gly Leu Glu Gln Asp Glu Thr Tyr Ile Pro Pro Gly Glu Ser Leu
   165 170 175
   Arg Asp Leu Ile Glu Gln Ser Gln Ser Ser Gly Ser Gly Ser Gly Leu
   180 185 190
   Pro Leu Leu Val Gln Arg Thr Ile Ala Lys Gln Ile Gln Met Val Lys
   195 200 205
   Gln Ile Gly Lys Gly Arg Tyr Gly Glu Val Trp Met Gly Lys Trp Arg
   210 215 220
   Gly Glu Lys Val Ala Val Lys Val Phe Phe Thr Thr Glu Glu Ala Ser
   225 230 235 240
   Trp Phe Arg Glu Thr Glu Ile Tyr Gln Thr Val Leu Met Arg His Glu
   245 250 255
   Asn Ile Leu Gly Phe Ile Ala Ala Asp Ile Lys Gly Thr Gly Ser Trp
   260 265 270
   Thr Gln Leu Tyr Leu Ile Thr Asp Tyr His Glu Asn Gly Ser Leu Tyr
   275 280 285
   Asp Tyr Leu Lys Ser Thr Thr Leu Asp Ala Lys Ser Met Leu Lys Leu
   290 295 300
   Ala Tyr Ser Ser Val Ser Gly Leu Cys His Leu His Thr Glu Ile Phe
   305 310 315 320
   Ser Thr Gln Gly Lys Pro Ala Ile Ala His Arg Asp Leu Lys Ser Lys
   325 330 335
   Asn Ile Leu Val Lys Lys Asn Gly Thr Cys Cys Ile Ala Asp Leu Gly
   340 345 350
   Leu Ala Val Lys Phe Ile Ser Asp Thr Asn Glu Val Asp Ile Pro Pro
   355 360 365
   Asn Thr Arg Val Gly Thr Lys Arg Tyr Met Pro Pro Glu Val Leu Asp
   370 375 380
   Glu Ser Leu Asn Arg Asn His Phe Gln Ser Tyr Ile Met Ala Asp Met
   385 390 395 400
   Tyr Ser Phe Gly Leu Ile Leu Trp Glu Val Ala Arg Arg Cys Val Ser
   405 410 415
   Gly Gly Ile Val Glu Glu Tyr Gln Leu Pro Tyr His Asp Leu Val Pro
   420 425 430
   Ser Asp Pro Ser Tyr Glu Asp Met Arg Glu Ile Val Cys Ile Lys Lys
   435 440 445
   Leu Arg Pro Ser Phe Pro Asn Arg Trp Ser Ser Asp Glu Cys Leu Arg
   450 455 460
   Gln Met Gly Lys Leu Met Thr Glu Cys Trp Ala His Asn Pro Ala Ser
   465 470 475 480
   Arg Leu Thr Ala Leu Arg Val Lys Lys Thr Leu Ala Lys Met Ser Glu
   485 490 495
   Ser Gln Asp Ile Lys Leu Asn Val Tyr Ile Met Ala Asp Lys Gln Lys
   500 505 510
   Asn Gly Ile Lys Ala Asn Phe Lys Ile Arg His Asn Ile Glu Asp Gly
   515 520 525
   Gly Val Gln Leu Ala Asp His Tyr Gln Gln Asn Thr Pro Ile Gly Asp
   530 535 540
   Gly Pro Val Leu Leu Pro Asp Asn His Tyr Leu Ser Phe Gln Ser Ala
   545 550 555 560
   Leu Ser Lys Asp Pro Asn Glu Lys Arg Asp His Met Val Leu Leu Glu
   565 570 575
   Phe Val Thr Ala Ala Gly Ile Thr Leu Gly Met Asp Glu Leu Tyr Lys
   580 585 590
   Val Asp Gly Gly Ser Gly Gly Thr Gly Val Ser Lys Gly Glu Glu Leu
   595 600 605
   Phe Thr Gly Val Val Pro Ile Leu Val Glu Leu Asp Gly Asp Val Asn
   610 615 620
   Gly His Lys Phe Ser Val Ser Gly Glu Gly Glu Gly Asp Ala Thr Tyr
   625 630 635 640
   Gly Lys Leu Thr Leu Lys Leu Ile Cys Thr Thr Gly Lys Leu Pro Val
   645 650 655
   Pro Trp Pro Thr Leu Val Thr Thr Phe Gly Tyr Gly Leu Lys Cys Phe
   660 665 670
   Ala Arg Tyr Pro Asp His Met Lys Gln His Asp Phe Phe Lys Ser Ala
   675 680 685
   Met Pro Glu Gly Tyr Val Gln Glu Arg Thr Ile Phe Phe Lys Asp Asp
   690 695 700
   Gly Asn Tyr Lys Thr Arg Ala Glu Val Lys Phe Glu Gly Asp Thr Leu
   705 710 715 720
   Val Asn Arg Ile Glu Leu Lys Gly Ile Asp Phe Lys Glu Asp Gly Asn
   725 730 735
   Ile Leu Gly His Lys Leu Glu Tyr Asn Gly Thr Gly Met Gly Val Gln
   740 745 750
   Val Glu Thr Ile Ser Pro Gly Asp Gly Arg Thr Phe Pro Lys Arg Gly
   755 760 765
   Gln Thr Cys Val Val His Tyr Thr Gly Met Leu Glu Asp Gly Lys Lys
   770 775 780
   Phe Asp Ser Ser Arg Asp Arg Asn Lys Pro Phe Lys Phe Met Leu Gly
   785 790 795 800
   Lys Gln Glu Val Ile Arg Gly Trp Glu Glu Gly Val Ala Gln Met Ser
   805 810 815
   Val Gly Gln Arg Ala Lys Leu Thr Ile Ser Pro Asp Tyr Ala Tyr Gly
   820 825 830
   Ala Thr Gly His Pro Gly Ile Ile Pro Pro His Ala Thr Leu Val Phe
   835 840 845
   Asp Val Glu Leu Leu Lys Leu Glu
   850 855